# FASCICULE DE BREVET EUROPEEN

(11) **EP 0 461 039 B1**
(45) Date de publication et mention de la délivrance du brevet: **16.09.1998**
(21) Numéro de dépôt: 91401480.8
(22) Date de dépôt: 06.06.1991
(51) Int. Cl.: C07D 403/10, C07D 235/08, C07C 229/38, C07D 257/04, C07C 233/54, C07C 233/43, C07C 233/40, C07C 233/25, C07C 233/15, A61K 31/41

(54) **Dérivés du benzimidazole, leur procédé de préparation, les intermédiaires obtenus, leur application à titre de médicaments et les compositions pharmaceutiques les renfermant**
Benzimidazol-Derivate, Verfahren und Zwischenprodukte zu ihrer Herstellung, ihre Anwendung als Arzneimittel und diese enthaltende pharmazeutische Zusammensetzungen
Benzimidazole derivatives, process and intermediates for their preparation, their use as medicaments and pharmaceutical compositions containing them

(30) Priorité: 08.06.1990 FR 9007135; 18.12.1990 FR 9015811; 28.03.1991 FR 9103778
(43) Date de publication de la demande: 11.12.1991
(73) Titulaire: HOECHST MARION ROUSSEL, 92800 Puteaux (FR)
(72) Inventeur: Fortin, Michel, F-75018 Paris (FR); Frechet, Daniel, F-75015 Paris (FR); Hamon, Gilles, F-93340 Le Raincy (FR); Jouquey, Simone, F-75020 Paris (FR); Vevert, Jean-Paul, F-93500 Pantin (FR)
(74) Mandataire: Vieillefosse, Jean-Claude

(56) Documents cités:
- EP-A- 0 186 190
- EP-A- 0 253 310
- EP-A- 0 392 317
- EP-A- 0 399 732
- EP-A- 0 400 835
- EP-A- 0 420 237
- EP-A- 0 425 921
- US-A- 4 880 804
- BEILSTEINS HANDBUCH DER ORGANISCHEN CHEMIE tome 23, supplément III/IV, 2ème partie, 4ème édition, 1980, Springer Verlag, Berlin, DE;
- BEILSTEINS HANDBUCH DER ORGANISCHEN CHEMIE tome 23, supplément III/IV, 4ème partie, 1980, Springer Verlag, Berlin, DE;
- BEILSTEINS HANDBUCH DER ORGANISCHEN CHEMIE tome 12, 4ème édition, 1929, Verlag von Julius Springer, Berlin, DE;
- BEILSTEINS HANDBUCH DER ORGANISCHEN CHEMIE tome 12, supplément II, 4ème édition, 1950, Springer Verlag, Berlin, DE;
- BEILSTEINS HANDBUCH DER ORGANISCHEN CHEMIE tome 12, supplément III, 2ème partie, 4éme édition, 1972, Springer Verlag, Berlin, DE;
- BEILSTEINS HANDBUCH DER ORGANISCHEN CHEMIE tome 12, supplément III, 4ème partie, 4ème édition, 1972, Springer Verlag, Berlin, DE;
- BEILSTEINS HANDBUCH DER ORGANISCHEN CHEMIE tome 13, supplément III, 2ème partie, 4ème édition, 1973, Springer Verlag, Berlin, DE;
- BEILSTEINS HANDBUCH DER ORGANISCHEN CHEMIE tome 14, supplément III, 2ème partie, 4ème édition, 1973, Springer Verlag, Berlin, DE;
- BEILSTEINS HANDBUCH DER ORGANISCHEN CHEMIE tome 12, supplément IV, 1ère partie, 4ème édition, 1984, Springer Verlag, Berlin, DE;
- BEILSTEINS HANDBUCH DER ORGANISCHEN CHEMIE tome 13, supplément IV, 2ème partie, 4ème édition, 1985, Springer Verlag, Berlin, DE;
- BEILSTEINS HANDBUCH DER ORGANISCHEN CHEMIE tome 14, supplément IV, 1ère partie, 4ème édition, 1985, Springer Verlag, Berlin, DE;
- BEILSTEINS HANDBUCH DER ORGANISCHEN CHEMIE tome 14, supplément IV, 2ème partie, 4ème édition, 1985, Springer Verlag, Berlin, DE;
- JOURNAL OF THE CHEMICAL SOCIETY Perkin Trans I, no. 7, 1979, pages 2289-2293; S.B. LANDOR et al.: "Allenes. PArt 38. Imidazolines, etc."
- CHEMICAL ABSTRACTS tome 90, no. 15, 9 avril 1975, page 567, colonne 1, abrégé no. 120660v, Columbus, Ohio, US; E.B. COLE et al.: "Photochemistry of heterocyclics" & Australien Journal of Chemistry 1979, tome 31, no. 12, pages 2675-2684
- CHEMICAL ABSTRACTS tome 95, no. 5, 3 août 1981, page 145, colonne 1, abrégé no. 36236g, Columbus, Ohio, US; V.M. REDDY et al.: "Laboratory evaluation of benzimidazoles against some phytopathogenic fungi." & Indian Drugs Pharm. Ind. 1980, tome 15, no. 4, pages 7-9
- CHEMICAL ABSTRACTS tome 74, no. 13, 29 mars 1971, page 241, colonne 2, abrégé no. 63452x, Columbus, Ohio, US; R.H. GUY: "Repellency of selected compounds to Tribolium castaneum" & J. Econ. Entomol. 1970, tome 63, no. 6, pages 1947-1850
- CHEMICAL ABSTRACTS tome 70, no. 5, 3 février 1969, page 1956, colonne 1, abrégé no. 19712a, Columbus, Ohio, US; L. RADZIUNAS et al.: "Acylation of vinyl ethers of aminophenols, and hydrogenation of the resulting products" & Liet. TSR Aukstr. Mokyklu Moskslo Darb., Chem. Chem. Technol. 1967, no. 8, pages 79-84
- CHEMICAL ABSTRACTS tome 91, no. 15, 8 octobre 1979, page 607, colonne 1, abrégé 123700q, Columbus, Ohio, US; B.P. JOSHIet al.: "Studies in quinazolones: Part I." & Indian Journal of Chemistry 1978, sect. B, tome 16B, no. 12, pages 1067- 1072
- JOURNAL OF ORGANIC CHEMISTRY tome 43, no. 4, 1978, pages 731-734, H.M. WALBORSKY et al.: "Alpha Addition and Ortho Metalation of Phenyl Isocyanide"
- JOURNAL OF THE CHEMICAL SOCIETY, CHEMICAL COMMUNICATIONS 1987, pages 1528-1530; S.J.F. MACDONALD et al.: "Synthesis of the Aglycones of the Ravidomycin Family of Antiboitics"

## Description

La présente invention concerne des dérivés de benzimidazole, leur procédé de préparation, les intermédiaires obtenus, leur application à titre de médicaments et les compositions pharmaceutiques les renfermant.

La présente invention a pour objet les produits de formule (I_{B}): 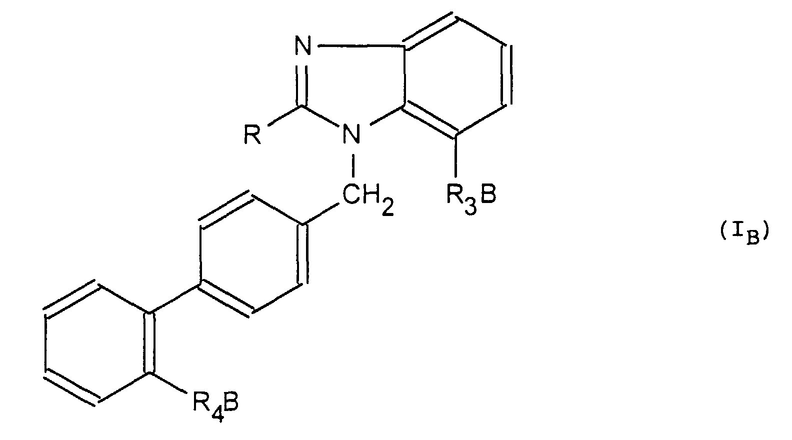 dans laquelle :
R représente un radical butyle ;
R_{3B} représente un radical -COOR₇ dans lequel R₇ représente un atome d'hydrogène ou un radical alkyle ou alkényle linéaire ou ramifié renfermant au plus 5 atomes de carbone ;
R_{4B} représente un radical carboxy libre, estérifié ou salifié; lesdits produits de formule (I_{B}) étant sous toutes les formes isomères possibles racémiques, énantiomères et diastéréoisomères, ainsi que les sels d'addition avec les acides minéraux ou organiques et les bases minérales ou organiques desdits produits de formule (I_{B}).

Selon un mode de réalisation, R_{3B} est COOH.
- Le document Beilstein, t23, suppl. III/IV, 2ème part., 1980, pp 1175, 1176 et 1188 décrit des dérivés alkylés de benzimidazoles ; il n'y a aucune divulgation d'un substituant biphényl méthyl.
- Le document Beilstein, t23, suppl. III/IV, 4ème part., 1980, pp 2564, 2565 et 2568 décrit des dérivés de benzimidazole ; il n'y a aucune divulgation d'un substituant biphénylméthyl.
- Le document Beilstein, t12, 4ème Ed., 1929, pp 794 et 795 décrit des dérivés de toluydyle.
- Le document Beilstein, t12, suppl. II, 4ème Ed., 1950, pp 147, 149 et 441 décrit des dérivés d'anilide.
- Le document Beilstein, t12, suppl. III, 2ème part., 1972, pp 488-490, décrit des dérivés d'anilide.
- Le document Beilstein, t12, suppl. III, 4ème part., 1972, pp 1850-1855 décrit des dérivés de toluydyle.
- Le document Beilstein, t13, suppl. III, 2ème part., 1973, pp 785-787 et 791 décrit des dérivés d'anilide.
- Le document Beilstein, t14, suppl. III, 2ème part., 1973, pp 922, 924-926 décrit des dérivés anthraniliques.
- Le document Beilstein, t12, suppl. IV, 1ère part., 1984, pp 391 et 392 décrit des dérivés d'anilide.
- Le document Beilstein, t13, suppl. IV, 2ème part., 1985, pp 837 et 838 décrit des dérivés d'anisidyle et de phénétydyle.
- Le document Beilstein, t14, suppl. IV, 1ère part., 1985, pp 44 et 45 décrit des dérivés d'anilide.
- Le document Beilstein, t14, suppl. IV, 2ème part., 1985, pp 1043 et 1044 décrit des dérivés anthraniliques.
- J. Chem. Soc. Perkin I, N° 7, 1979, pp 2289-2293 décrit des dérivés alkylés de benzimidazole ; il n'y a aucune mention d'un substituant biphénylméthyl.
- Le document CAS, 1979, 90, Abs N° 120660v décrit des dérivés alkylés de benzimidazole sans mention d'un substituant biphénylméthyl.
- Le document CAS, 1981, 95, Abs N° 36236g décrit des dérivés alkylés de benzimidazole sans mention d'un substituant biphénylméthyl.
- Le document CAS, 1971, 74, Abs N° 63452x décrit des dérivés de benzotriazines.
- Le document CAS, 1969, 70, Abs N° 19712a décrit des dérivés d'aminophénol.
- Le document CAS, 1979, 91, Abs N° 123700q décrit des dérivés de quinazolones.
- J. Org. Chem., 1978, 43, pp 731-734 décrit des dérivés de phénylisocyanuse.
- J. Chem. Soc. Chem. Commun., 1987, pp 1528-1530 décrit des dérivés de quinone.
Aucun des documents ci-dessus ne décrit les présents dérivés de benzimidazole, ni leur activité pharmacologique.
- US-A-4880804 (Carini et al) décrit des dérivés de benzimidazole présentant un substituant biphénylméthyl, un substituant alkyle en position 2 et un substituant fonctionnel en position 6. Ces composés présentent une activité inhibitrice de l'angiotensine II.
- EP-A-0253310 décrit des dérivés imidazoles.
- EP-A-0186190 décrit des dérivés de (thio)-phénoxylbenzylméthylbenzimidazole ; il n'y a pas de mention du groupe biphényl selon l'invention.
- EP-A-0420237 décrit un dérivé de biphénylméthylbenzimidazole, dans l'exemple 1, ce dérivé ne portant aucun substituant en position 7.
- EP-A-0425921 décrit des dérivés de biphénylméthylbenzimidazole, dans lesquels le phényl distal du groupe biphényl porte un substituant tétrazolyle.
- EP-A-0392317 décrit des dérivés de biphénylméthylbenzimidazole, dans lesquels divers substituants sont présents sur le cycle. Il n'y a aucune mention de la substitution en position 7 par un groupe acide carboxylique ou un dérivé.
- EP-A-0399732 décrit des dérivés de biphénylméthylbenzimidazole, dans lesquels divers substituants sont présents sur le cycle. Il n'y a aucune mention de la substitution en position 7 par un groupe acide carboxylique ou un dérivé.
- EP-A-0400835 décrit des dérivés de biphénylméthylbenzimidazole, dans lesquels divers substituants sont présents sur le cycle. Il n'y a aucune mention de la substitution en position 7 par un groupe acide carboxylique ou un dérivé.

Dans les produits de formule (I_{B}), et dans ce qui suit :
- le terme radical alkyle linéaire ou ramifié désigne de préférence les radicaux méthyle, éthyle, propyle ou isopropyle, butyle, isobutyle, sec-butyle ou tert-butyle mais peut également représenter un radical pentyle et en particulier tert-pentyle,
- le terme radical alkényle linéaire ou ramifié désigne de préférence un radical vinyle, allyle, 1-propényle, 2-propényle, 1-butényle, 2-butényle mais peut également représenter un radical 1-pentényle, 2-pentényle ou encore 3-pentényle,

On peut citer pour les radicaux carboxy estérifiés, de préférence un groupe (alkyloxy ou alkényloxy inférieur carbonyle) tel que méthoxycarbonyle ou éthoxycarbonyle mais également les radicaux propoxycarbonyle, butoxycarbonyle, butényloxycarbonyle, tert-butoxycarbonyle, ou encore pentoxycarbonyle.

Les sels d'addition avec les acides minéraux ou organiques et les bases minérales ou organiques des produits de formule (I_{B}) peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, ascorbique, les acides alkylmonosulfoniques tels que par exemple l'acide méthanesulfonique, l'acide éthanesulfonique, l'acide propanesulfonique, les acides alkyldisulfoniques tels que par exemple l'acide méthanedisulfonique, l'acide 1,2-éthanedisulfonique, les acides arylmonosulfoniques tels que l'acide benzènesulfonique et les acides aryldisulfoniques.

On préfère notamment les sels formés avec l'acide chlorhydrique.

Le ou les radicaux carboxy des produits de formule (I_{B}) peuvent être salifiés par des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine.

Parmi les produits objet de l'invention, peut être cité tout particulièrement :
- l'acide 2-butyl 1-[(2'-carboxy biphényl-4-yl) méthyl] 1H-benzimidazole-7-carboxylique et ses sels,

L'invention a également pour objet un procédé de préparation de produits de formule (I_{B}) caractérisé en ce que : **soit** on fait réagir un produit de formule (IV_{B}) : 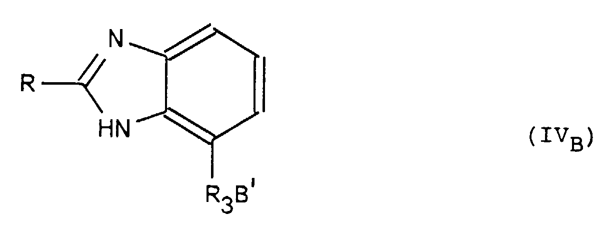 dans laquelle R et R_{3B}' ont les significations indiquées à la revendication 1, respectivement pour R et R_{3B} dans lesquelles les éventuelles fonctions réactives sont éventuellement protégées par des groupements protecteurs, avec un composé de formule (V_{B}) : 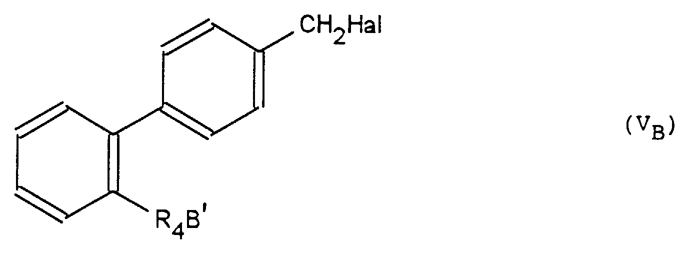 dans laquelle Hal représente un atome d'halogène et R_{4B}' a la signification indiquée à la revendication 1 pour R_{4B} dans lequel les éventuelles fonctions réactives sont éventuellement protégées par des groupements protecteurs, pour obtenir des produits de formule (IX_{B}) : 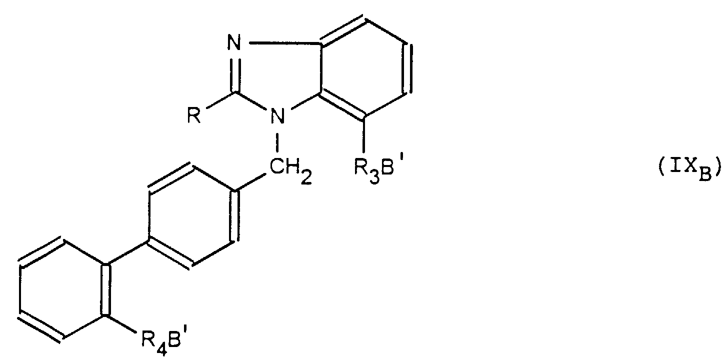 dans laquelle R, R_{3B}' et R_{4B}' ont les significations indiquées ci-dessus,
**soit** a) l'on fait réagir un composé de formule (VI_{B}) : 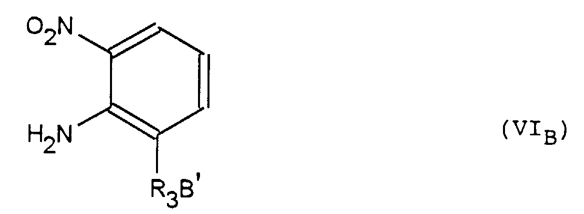 dans laquelle R_{3B}' a la signification précédente, ou bien avec le composé de formule (II) : 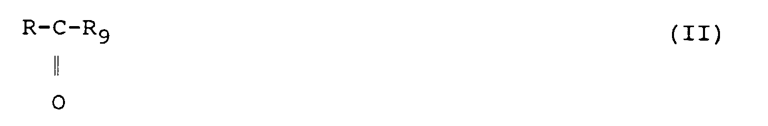 dans laquelle R₉ représente un radical hydroxy, alkyloxy ou un atome d'halogène et R a la signification indiquée ci-dessus, pour obtenir le produit de formule (X_{B}) : 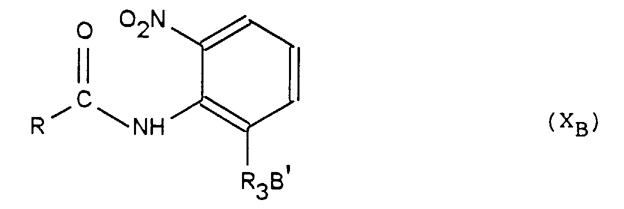 dans laquelle R et R_{3B}' ont les significations précédentes,
b) l'on fait réagir un composé de formule (VI_{B}') : 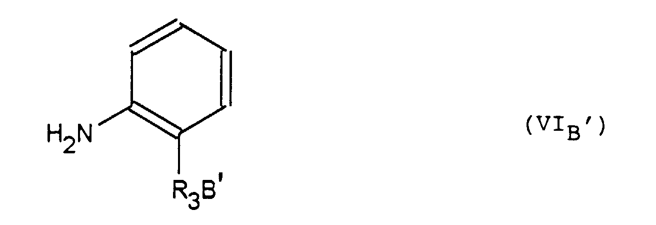 dans laquelle R_{3B}' a la signification précédente, avec le composé de formule (II) tel que défini ci-dessus pour obtenir le produit de formule (X_{B}') : 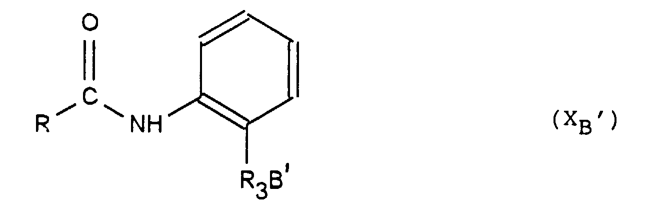 dans laquelle R_{3B}' et R ont les significations indiquées précédemment,
   que l'on soumet à une réaction de nitration pour obtenir le composé de formule (X_{B}) tel que défini ci-dessus,
   produit de formule (X_{B}) que l'on fait réagir avec le composé de formule (V_{B}) telle que définie ci-dessus, pour obtenir un produit de formule (XI_{B}) : 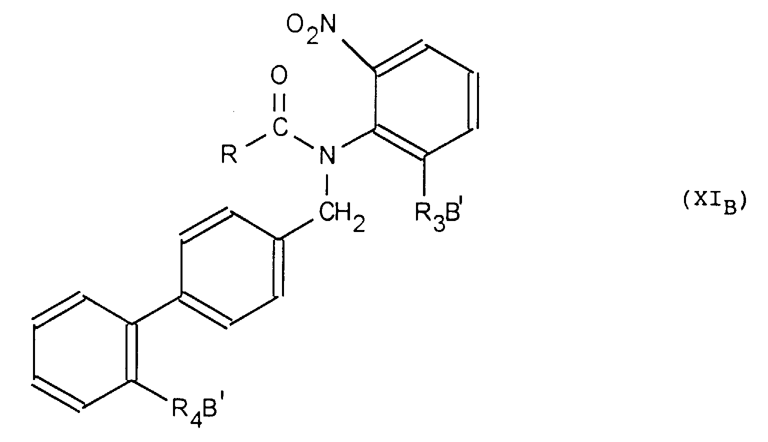 dans laquelle R, R_{3B}' et R_{4B}' ont les significations précédentes, que l'on soumet à une réaction de réduction sélective de la fonction nitro, pour obtenir le produit de formule (XII_{B}) : 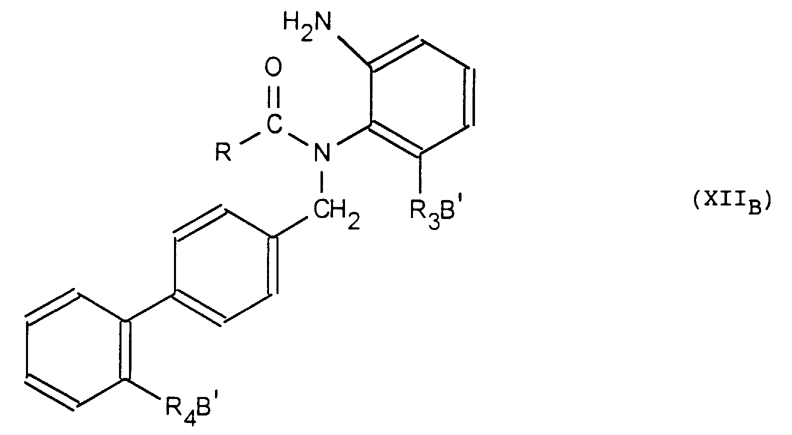 dans laquelle R, R_{3B}' et R_{4B}' ont les significations précédentes, que l'on soumet à une réaction de cyclisation pour obtenir des produits de formule (IX_{B}) telle que définie ci-dessus,
   **soit** l'on fait réagir le composé de formule (VI_{B}) avec le composé de formule (V_{B}) telle que définie ci-dessus, pour obtenir des produits de formule (VII_{B}) : 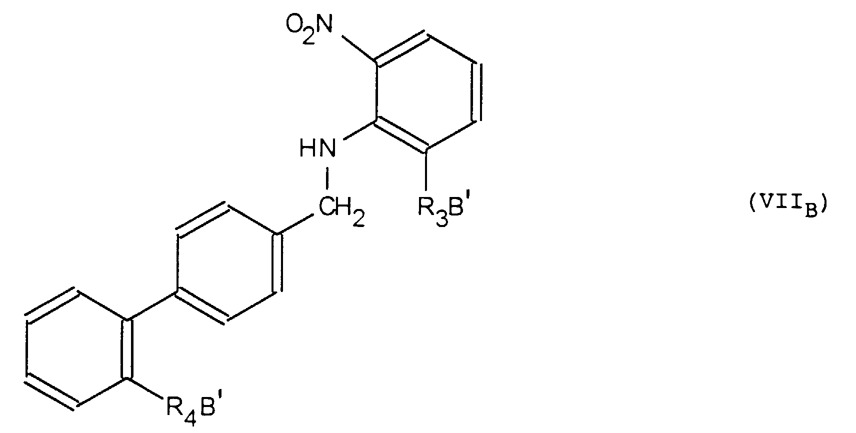 dans laquelle R_{3B}' et R_{4B}' ont les significations indiquées ci-dessus, que l'on soumet à une réaction de réduction du radical nitro en radical amino pour obtenir des produits de formule (VIII_{B}) : 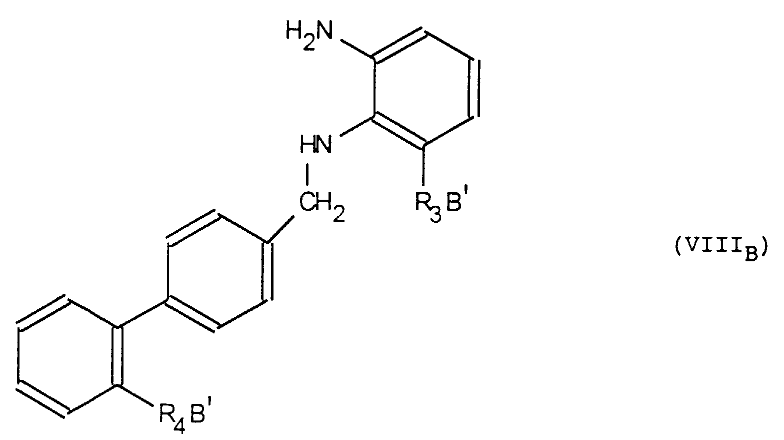 dans laquelle R_{3B}' et R_{4B}' ont les significations indiquées ci-dessus, que l'on fait réagir avec le produit de formule (III) : 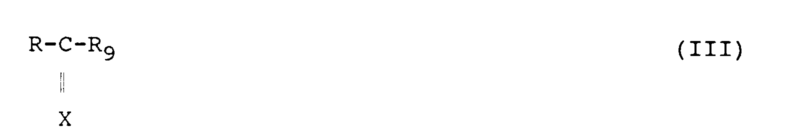 dans laquelle X représente un atome d'oxygène ou un radical NH, R₉ et R ont la signification indiquée ci-dessus, pour obtenir après cyclisation, un produit de formule (IX_{B}) telle que définie ci-dessus,
   produit de formule (IX_{B}) que l'on soumet, si désiré et si nécessaire, à l'une ou plusieurs des réactions suivantes, dans un ordre quelconque :
   - une réaction d'élimination des groupements protecteurs que peuvent porter les fonctions réactives protégées,
   - une réaction de salification par un acide ou une base minéral ou organique pour obtenir le sel correspondant,
   - une réaction d'estérification ou salification d'une fonction acide,
   - une réaction d'hydrolyse acide ou alcaline d'une fonction ester en fonction acide,
   - une réaction de dédoublement des formes racémiques en produits dédoublés,
   lesdits produits de formule (I_{B}) ainsi obtenus étant sous toutes les formes isomères possibles racémiques, énantiomères et diastéréoisomères.

Dans des conditions préférentielles de mise en oeuvre de l'invention, le procédé ci-dessus est réalisé de la manière suivante :

La réaction du composé de formule (V_{B}) dans laquelle l'atome d'halogène représente de préférence un atome de brome sur l'anion de l'imidazole de formule (IV_{B}) préparé par exemple par action d'un agent alcalin tel que l'hydrure de sodium ou de potassium ou encore d'un alcoolate de sodium ou de potassium tel que par exemple le méthylate de sodium, peut être réalisée, par exemple, dans un solvant organique tel que le diméthylformamide ou le tétrahydrofuranne.

La réaction d'addition des composés de formule (II) sur la fonction amine libre des composés de formule (VI_{B}) ou (VI_{B}') pour obtenir respectivement les produits de formule (X_{B}) ou (X_{B}') peut être réalisée par simple chauffage à une température d'environ 120°C à 170°C : un composé de formule (II) peut être par exemple l'acide valérique, utilisé alors, de préférence en excès par rapport au composé de formule (VI_{B}) ou (VI_{B}').

La réaction de nitration des produits de formule (X_{B}') pour donner les produits de formule (X_{B}) peut être réalisée par exemple en présence d'acide nitrique dans un solvant tel que par exemple l'anhydride acétique et l'acide acétique à une température comprise entre 0 et 10°C.

La réaction d'addition des composés de formule (V_{B}) sur la fonction amide des composés de formule (X_{B}) pour obtenir les produits de formule (XI_{B}) peut être réalisée à la température ambiante ou par chauffage à une température comprise entre 20°C et 150°C, de préférence en présence d'un agent alcalin tel que par exemple la triéthylamine, la soude, le méthylate ou l'éthylate de sodium ou encore l'hydrure de sodium dans un solvant tel que par exemple le tétrahydrofuranne ou le diméthylformamide.

La réduction du radical nitro des produits de formule (XI_{B}) en radical amino pour obtenir les produits de formule (XII_{B}) peut être réalisée selon les méthodes usuelles connues de l'homme du métier notamment par une hydrogénation catalytique en présence d'hydroxyde de palladium dans un solvant tel que par exemple l'éthanol ou par du zinc dans un solvant tel que par exemple l'acide acétique en présence d'acétate de sodium ou encore par du borohydrure de sodium.

La réaction de cyclisation des produits de formule (XII_{B}) pour obtenir les produits de formule (IX_{B}) peut être réalisée par simple chauffage ou en présence d'un catalyseur tel que par exemple le chlorure de thionyle, le pentachlorure de phosphore ou encore l'anhydride phosphorique dans un solvant tel que par exemple le tétrahydrofuranne ou le diméthylformamide.

La réaction d'addition des composés de formule (V_{B}) sur la fonction amine libre des composés de formule (VI_{B}) pour obtenir les produits de formule (VII_{B}) peut être réalisée dans les mêmes conditions que celles décrites ci-dessus pour l'addition des composés de formule (V_{B}) sur les produits de formule (IV_{B}).

La réduction du radical nitro des produits de formule (VII_{B}) en radical amino pour obtenir les produits de formule (VIII_{B}) peut être réalisée selon les méthodes usuelles connues de l'homme du métier notamment dans les mêmes conditions que celles décrites ci-dessus pour la réduction du radical nitro des produits de formule (XI_{B}).

La réaction d'addition des composés de formule (III) sur le radical amine libre des produits de formule (VIII_{B}) suivie de la cyclisation des produits ainsi obtenus peut être réalisée selon diverses conditions réactionnelles connues de l'homme du métier, de préférence dans un solvant organique tel que par exemple le tétrahydrofuranne ou le diméthylformamide à une température comprise entre 20°C et 200°C.

Le composé de formule (III) peut être, par exemple, quand X représente NH, le pentanimidoate d'éthyle et quand X représente O, par exemple, l'acide valérique.

Selon les valeurs de R_{3B}' et R_{4B}', les produits de formule (IX_{B}) constituent ou non des produits de formule (I_{B}).

Les diverses fonctions réactives que peuvent porter certains composés définis ci-dessus peuvent, si nécessaire, être protégées : il s'agit par exemple des radicaux carboxy libres qui peuvent être protégés par les groupes protecteurs appropriés facilement éliminables.

La liste suivante, non exhaustive, d'exemples de protection de fonctions réactives peut être citée :
les groupes carboxy peuvent être protégés par exemple sous forme d'esters formés avec les esters facilement clivables tels que les esters benzyliques ou tert-butyliques ou des esters connus dans la chimie des peptides.

L'élimination de ces groupes protecteurs peut être effectuée dans les conditions usuelles connues de l'homme du métier notamment par hydrolyse acide effectuée avec un acide tel que les acides chlorhydrique, benzènesulfonique, para-toluènesulfonique, formique ou trifluoroacétique.

On trouvera une liste de différents groupes protecteurs utilisables par exemple dans le brevet BF 2 499 995.

Les produits décrits ci-dessus peuvent, si désiré, faire l'objet de réactions de salification par un acide minéral ou organique réalisées selon les méthodes usuelles connues de l'homme du métier.

Les éventuelles formes optiquement actives des produits de formule (I_{B}) peuvent être préparées par dédoublement des racémiques selon les méthodes usuelles connues de l'homme du métier. On peut par exemple utiliser les sels formés avec des bases optiquement actives.

Les composés de formule (I_{B}) tels que définis ci-dessus ainsi que leurs sels d'addition avec les acides présentent d'intéressantes propriétés pharmacologiques.

Les produits sont doués de propriétés antagonistes pour le récepteur à l'angiotensine II et sont ainsi notamment inhibiteurs des effets de l'angiotensine II, en particulier de l'effet vasoconstricteur et également de l'effet trophique au niveau des myocytes.

Ces propriétés justifient leur application en thérapeutique et l'invention a également pour objet à titre de médicaments, les produits tels que définis par la formule (I_{B}) ci-dessus, lesdits produits de formule (I_{B}) étant sous toutes les formes isomères possibles racémiques ou optiquement actives, ainsi que les sels d'addition avec les acides minéraux ou organiques et les bases minérales ou organiques pharmaceutiquement acceptables desdits produits de formule (I_{B}).

L'invention a plus particulièrement pour objet, à titre de médicaments, le produit de formule (I_{B}) suivant :
- l'acide 2-butyl 1-[[2'-carboxy biphényl-4-yl] méthyl] 1H-benzimidazole-7-carboxylique,
ainsi que ses sels d'addition avec les acides minéraux ou organiques et les bases minérales ou organiques pharmaceutiquement acceptables.

Les médicaments, objet de l'invention, peuvent être utilisés dans le traitement de l'hypertension artérielle, des insuffisances cardiaques, des insuffisances rénales et dans la prévention des resténoses post-angioplastie.

Ils peuvent également être utilisés dans le traitement de certains désordres gastro-intestinaux, gynécologiques et en particulier pour leur effet relaxant au niveau de l'utérus.

L'invention s'étend aux compositions pharmaceutiques renfermant à titre de principe actif l'un au moins des médicaments tels que définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous toutes les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 1 à 100 mg par jour chez l'adulte, par voie orale.

Les composés de départ de formules (II), (III), (IV_{B}), (V_{B}), (VI_{B}) et (VI_{B}') peuvent être disponibles dans le commerce ou peuvent être préparées selon les méthodes usuelles connues de l'homme du métier.

La préparation de certains composés de formule (IV_{B}) est décrite dans : J. Amer. Chem. Soc. (1937) 59, 178.

Les autres produits de formule (IV_{B}) peuvent être préparés par action d'un composé de formule (III) telle que définie ci-dessus avec un produit de formule (XIII) : 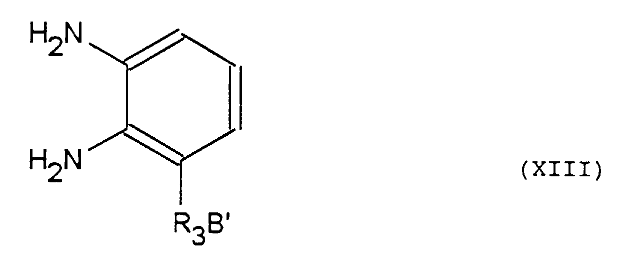 dans laquelle R_{3B}' a la signification indiquée ci-dessus.

Les conditions opératoires sont les mêmes que celles indiquées ci-dessus pour l'action des produits de formule (III) sur les produits de formule (VIII_{B}).

Certains composés de formule (XIII) peuvent être trouvés dans le commerce comme par exemple le 3,4-diamino benzoate de méthyle, commmercialisé par exemple par LANCASTER.

Un exemple de préparation de ces composés de formule (XIII) est donné dans :
Journal of the Chemical Society, Chemical Communications, (1957) 2197-2201.

Parmi les composés de formules (II) et (III) se trouve par exemple le pentanimidoate d'éthyle qui peut être préparé, par exemple, par action d'acide chlorhydrique gazeux dans l'éthanol sur le valéronitrile, commmercialisé par exemple, par LONZA.

Un exemple de préparation de ces composés de formule (III) est donné dans J. Amer. Chem. Soc. (1942), 64, 1827.

Un procédé de préparation des produits de formule (V_{B}) telle que définie ci-dessus peut consister à soumettre le iodobenzoate de méthyle, commercialisé par exemple, par JANSSEN, à l'action du iodotoluène, commercialisé par exemple, par FLUKA, la réaction est réalisée par exemple en présence de cuivre en poudre à une température de 100°C à 300°C, pour obtenir un produit de formule (V_{B}') : 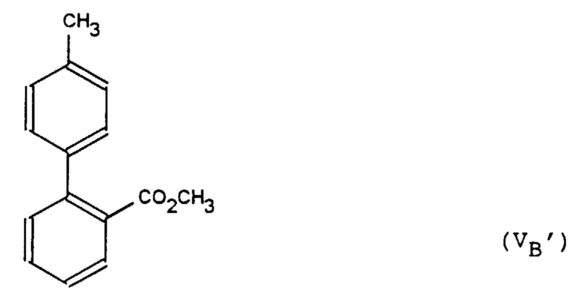 dont le radical carboxy estérifié peut, si désiré, être libéré du radical alkyle par les méthodes classiques connues de l'homme du métier ou indiquées ci-dessus, par exemple l'hydrolyse acide ou alcaline, que l'on peut soumettre à une réaction de bromation sur le radical méthyle par les méthodes classiques connues de l'homme du métier par exemple par action du N-bromosuccinimide dans le tétrachlorure de carbone.

La préparation de certains composés de formule (V_{B}) ou (V_{B}') ou encore certaines transformations du radical R_{4B}' des produits de formule (IX_{B}) peuvent être trouvées, par exemple, dans le brevet US 4,880,804 ou EP 0 253 310.

Le composé de formule (VI_{B}) peut être par exemple l'orthonitroaniline, sous forme de produit commercialisé par exemple, par UCB.

Les produits de formule (VI_{B}) peuvent également être préparés, par exemple, par le procédé décrit dans : Canadian journal of chemistry, (1977), 55 (10), pp 1653-1657.

Les produits de formule (VI_{B}') qui sont des dérivés de l'aniline peuvent être trouvés dans le commerce tel que par exemple la 2,4-diméthoxy aniline chez ALDRICH ou peuvent être préparés ainsi qu'il est indiqué par exemple dans les références suivantes :
- BEILSTEIN volume XII, 3ème supplément p. 1662
- BEILSTEIN volume XIII, 3ème supplément p. 2128.

La présente invention a enfin pour objet à titre de produits intermédiaires nécessaires à la préparation des produits de formule (I_{B}), les composés de formules (VII_{B}) et (VIII_{B}).

En plus des produits décrits dans les exemples qui illustrent l'invention sans toutefois la limiter, les produits suivants constituent des produits pouvant être obtenus dans le cadre de la présente invention : ces produits de formule (I_{B}) sont tels que R représente un radical butyle, R_{4B} représente un radical carboxy et R_{3B} a la signification suivante COOMe. Les exemples suivants illustrent l'invention.

### PREPARATION 1 : 4'-[N-[2-(méthoxycarbonyl) 6-nitro phényl] N-(pentanoyl) aminométhyl] biphényle-2-carboxylate de méthyle

### STADE A : Acide 2-amino 3-nitro benzoïque

### a) acétylation :

A une suspension refroidie à +10°C de 12 g de 2-amino 3-nitro toluène et 14,4 cm³ d'anhydride acétique, on ajoute 0,65 cm³ d'acide sulfurique concentré, on agite pendant 1 heure à température ambiante, ajoute 50 cm³ d'eau, agite 15 minutes, essore, lave à l'eau puis à l'éther, sèche à 100°C sous pression réduite. On obtient 13,6 g de produit recherché. F = 156°C.

### b) oxydation :

A une suspension de 29,48 g de permanganate de potassium, 21,44 g de sulfate de magnésium dans 1470 cm³ d'eau, on ajoute 13,4 g du composé obtenu ci-dessus. On agite pendant 3 heures à 100°C, laisse revenir à température ambiante, refroidit à 0°C +5°C, filtre l'insoluble, au filtrat refroidi à 0°C +5°C, on ajoute 10 cm³ d'acide chlorhydrique concentré (pH 2-3). On extrait avec 4 fois 500 cm³ d'acétate d'éthyle, lave à l'eau, sèche, filtre et évapore sous pression réduite. On obtient 16,1 g de produit que l'on cristallise dans l'éther éthylique pour recueillir 12,1 g du produit recherché. F = 192°C.

### c) hydrolyse :

12 g du produit précédemment obtenu sont agités pendant 4 heures à 120°C avec 75 cm³ d'eau à 5 % d'acide chlorhydrique concentré, on laisse revenir à température ambiante, refroidit à 0°C +5°C, agite 1 heure à cette température, essore, lave à l'eau puis à l'éther, sèche à 100°C sous pression réduite. On obtient 8,8 g du produit recherché. F = 210°C.

### STADE B : 2-amino 3-nitro benzoate de méthyle

On agite pendant 48 heures, au reflux une suspension de 1 g de l'acide obtenu au stade A, 15 cm³ de méthanol et 1,4 cm³ d'acide sulfurique à 95-97 %. On évapore le méthanol, ajoute 100 cm³ d'eau, alcalinise par ajout de bicarbonate de sodium, extrait avec 3 fois 200 cm³ de chlorure de méthylène, lave à l'eau, sèche, filtre et évapore sous pression réduite. On obtient 1 g de produit attendu. F = 96°C.

### STADE C : 3-nitro 2-pentanamido benzoate de méthyle

On agite pendant 1 heure au reflux 850 mg de produit obtenu au stade B ci-dessus, et 5 cm³ de chlorure de valéryle. On évapore à sec et reprend le résidu avec 80 cm³ d'éther éthylique, traite avec du charbon actif, filtre et évapore sous pression réduite. On obtient 1,3 g de produit que l'on cristallise dans un mélange d'éther isopropylique et de pentane, on recueille 1 g de produit recherché. F = 58°C.

Un échantillon analytique a été préparé par dissolution dans 20 cm³ d'éther isopropylique au reflux, filtration et concentration à 5 cm³. On laisse au repos 18 heures à température ambiante, essore, lave à l'éther isopropylique. On obtient 710 mg. F = 60°C.

On dissout ces 710 mg dans 25 cm³ d'éther isopropylique à chaud, traite avec du charbon actif, filtre, concentre à 10 cm³. On laisse au repos à température ambiante, pendant 1 heure, essore, lave à l'éther isopropylique. On obtient 380 mg du produit recherché. F = 60°C.

| Analyse pour C₁₃H₁₆N₂O₅ = 280,27 | | | |
|---|---|---|---|
| | C | H | N |
| % calculés | 55,71 | 5,75 | 10,00 |
| % trouvés | 55,8 | 5,38 | 9,9 |

| Spectre I.R. (CHCl₃) | |
|---|---|
| NH complexe | 3400 cm⁻¹ (ep) 3330 cm⁻¹ (max) |
| C=O complexe | 1730 cm⁻¹ (ep) 1708 cm⁻¹ (max) |
| Aromatique + Amide II | 1608 - 1588 - 1499 cm⁻¹ |
| NO₂ | 1540 - 1360 cm⁻¹ |

### STADE D :4'-[N-[2-(méthoxycarbonyl) 6-nitro phényl] N-(pentanoyl) aminométhyl] biphényle-2-carboxylate de méthyle

A une solution de 1 g du produit obtenu au stade B ci-dessus, dans 5 cm³ de diméthylformamide, on ajoute 170 mg d'hydrure de sodium à 50 % dans l'huile. On agite encore 5 minutes après la fin du dégagement d'hydrogène puis ajoute 1,09 g de 4'-(bromométhyl) biphényle-2-carboxylate de méthyle (préparation EP 00253310) on agite pendant 1 heure à température ambiante. On évapore le solvant et reprend le résidu par 3 fois 250 cm³ d'acétate d'éthyle puis lave avec 3 fois 50 cm³ d'eau, sèche, filtre et évapore sous pression réduite. On obtient 2 g de produit que l'on chromatographie sur silice (éluant : chlorure de méthylène-méthanol 99-1). On recueille 1,6 g du produit recherché.

| Spectre I.R. (CHCl₃) | |
|---|---|
| C=O complexe | 1726 et 1670 cm⁻¹ |
| NO₂ | 1538 et 1352 cm⁻¹ |
| Aromatiques | 1600 et 1576 cm⁻¹ |

### EXEMPLE 1 : chlorhydrate de 2-butyl 1-[2'-(méthoxycarbonyl) biphényl-4-yl] méthyl] 1H-benzimidazole-7-carboxylate de méthyle

### a) hydrogénation :

A une solution de 730 mg du composé obtenu au stade D de la préparation 1, dans 15 cm³ de tétrahydrofuranne, on ajoute 365 mg de palladium à 18 % sur charbon actif, on agite pendant 1 heure 30, sous atmosphère d'hydrogène (109 cm³ d'hydrogène sont absorbés) on filtre le catalyseur, rince avec du chlorure de méthylène et évapore à sec sous pression réduite, on obtient 730 mg de produit.

### b) cyclisation :

Les 730 mg de produit ci-dessus, sont dissous dans 5 cm³ d'acétate d'éthyle et 5 cm³ d'isopropanol et on ajoute un large excès d'une solution d'acide chlorhydrique dans l'acétate d'éthyle. On chauffe à 50°C pendant 5 minutes et laisse 30 minutes à température ambiante, on évapore les solvants et reprend le résidu avec 3 fois 3 cm³ d'acétate d'éthyle. On essore, lave à l'éther. On obtient 640 mg du produit recherché. F = 160°C.

190 mg du produit ci-dessus sont recristallisés dans l'isopropanol, on obtient 163 mg du produit attendu. F = 160°C.

| Analyse pour C₂₈H₂₈N₂O₄, HCl = 492,99 | | | | |
|---|---|---|---|---|
| | C | H | Cl | N |
| % calculés | 68,21 | 5,93 | 7,19 | 5,68 |
| % trouvés | 67,8 | 5,8 | 7,3 | 5,5 |

| Spectre I.R. | |
|---|---|
| Absorption complexe type N^{⊕}-H | 2380 cm⁻¹ |
| C=O | 1727 et 1721 cm⁻¹ |

### EXEMPLE 2 : Acide 2-butyl 1-[(2'-carboxy biphényl-4-yl) méthyl] 1H-benzimidazole-7-carboxylique

On agite 1 heure au reflux une solution de 440 mg du produit obtenu à l'exemple 3, avec 10 cm³ d'éthanol, 1 cm³ d'eau, 1 cm³ de lessive de soude. On évapore l'éthanol, dissout le résidu dans l'eau (10 cm³) et acidifie à pH 3-4 avec de l'acide acétique. Après essorage, on obtient 360 mg de produit F = 160°C, puis 250°C, on recristallise 450 mg de produit obtenu comme ci-dessus, dans 15 cm³ de méthanol, 5 gouttes d'acide acétique et 5 cm³ d'eau. On obtient 370 mg de produit F = 255°C, que l'on dissout dans 20 cm³ de méthanol et 10 cm³ de chlorure de méthylène au reflux, on filtre, évapore le chlorure de méthylène et ajoute 1 goutte d'acide acétique et 5 cm³ d'eau. Après une nuit à température ambiante, on essore et obtient 330 mg de produit. F = 255°C.

| Analyse pour C₂₆H₂₄N₂O₄ = 428,47 | | | |
|---|---|---|---|
| | C | H | N |
| % calculés | 72,88 | 5,65 | 6,54 |
| % trouvés | 73,0 | 5,6 | 6,5 |

| Spectre I.R. (nujol) | |
|---|---|
| Absorption générale région OH/NH | |
| C=O | 1704 cm⁻¹ |

| Spectre de RMN (DMSO) | |
|---|---|
| CH₃ | 0,88 (t) |
| CH₂ | 1,39 (m) |
| CH₂ | 1,76 (m) |
| CH₂-C= | 2,87 (m) |
| N-CH₂- | 5,90 (s) |
| C₆H₄ | 6,88 (d,l) 7,23 (d,l) |
| 7 autres H aromatiques | 7,20 à 7,90 (m) |
| H mobiles | ∼ 12,95 (m) |

### EXEMPLE 3 : composition pharmaceutique

On a préparé des comprimés répondant à la formule suivante :

| | |
|---|---|
| Produit de l'exemple 2 | 10 mg |
| Exipient pour un comprimé terminé à | 100 mg |
| (détail de l'exipient : lactose, talc, amidon, stéarate). | |

### RESULTATS PHARMACOLOGIQUES

### 1 - Test sur le récepteur de l'angiotensine II

On utilise une préparation membranaire fraîche obtenue à partir de foie de rat. Le tissu est broyé au polytron dans un tampon Tris 50 mM pH 7,4 le broyage est suivi de 3 centrifugations à 30 000 g 15' avec reprises intermédiaires des culots dans le tampon Tris pH 7,4.

Les derniers culots sont remis en suspension dans un tampon d'incubation (Tris 20 mM, NaCl 135 mM, KCl 10 mM, glucose 5 mM, MgCl₂ 10 mM, fluorure de phényl méthyl sulfonyle 0,3 mM, bacitracine 0,1 mM, bis(triméthylsilyl) acétamide 0,2 %).

On répartit des fractions aliquotes de 2 ml dans des tubes à hémolyse et ajoute de la ¹²⁵I angiotensine II (25 000 DPM/tube) et le produit à étudier. (Le produit est d'abord testé à 3 x 10⁻⁵M en triple). Lorsque le produit testé déplace de plus de 50 % la radioactivité liée spécifiquement au récepteur, il est testé à nouveau selon une gamme de 7 concentrations afin de déterminer la concentration qui inhibe de 50 % la radioactivité liée spécifiquement au récepteur. On détermine ainsi la concentration inhibitrice 50 %).

La liaison non spécifique est déterminée par addition d'un produit de référence soit le produit de l'exemple 94 du brevet européen 0253310 à 10⁻⁵M (en triple). On incube à 25°C pendant 150 minutes, remet au bain-marie à 0°C, 5 minutes, filtre sous vide, rince au tampon Tris pH 7,4 et compte la radioactivité en présence du scintillant Triton.

Le résultat est exprimé directement en concentration inhibitrice 50 % (CI₅₀), c'est-à-dire en concentration de produit étudié, exprimée en nM, nécessaire pour déplacer 50 % de la radioactivité spécifique fixée sur le récepteur étudié.

| Résultats : | |
|---|---|
| Produit de l'exemple | CI₅₀ en nanomoles |
| 2 | 52 |

### 2 - Mise en évidence de l'activité antagoniste de l'angiotensine II sur la veine porte isolée.

La veine porte est prélevée, sur des rats mâles Wistar (350 g environ) (IFFA Crédo Franc) après dislocation cervicale, et mise rapidement dans une solution physiologique (voir ci-dessous) à température ambiante. Un anneau de 1 mm environ est monté dans un bain à organe isolé contenant 20 ml de la solution physiologique suivante (composition en mM : NaCl 118,3 - KCl 4,7 - MgSO₄ 1,2 - KH₂PO₄ 1,2 - NaHCO₃ 25 - glucose 11,1 - CaCl2 2,5) le milieu est maintenu à 37°C et oxygéné par un mélange O₂ 95 %, CO₂ 5 %. La tension initiale imposée est de 1 g, les anneaux sont laissés au repos pendant 60 à 90 minutes. Afin d'éviter les contractions spontanées, du vérapamil est ajouté au bain d'incubation (1.10⁻⁶M).

A la fin de la période de repos l'angiotensine II (hypertensine Ciba) 3.10⁻⁸M est ajoutée dans le bain d'incubation et laissée au contact de la préparation pendant 1 minute. Cette opération est répétée chaque 30 minutes, le tissu étant lavé 3 ou 4 fois entre deux stimulations à l'angiotensine. Le composé à étudier est introduit dans le bain 15 minutes avant une nouvelle stimulation par l'angiotensine. Des concentrations croissantes de la molécule étant appliquées, une CI₅₀ (concentration qui produit une inhibition de 50 % de la réponse à l'angiotensine) peut être calculée, celle-ci est exprimée en nanomoles.

| Résultats : | |
|---|---|
| Produit de l'exemple | CI₅₀ en nanomoles |
| 2 | 9,5 |

### 3 - Test d'activité antagoniste de l'angiotensine II chez le rat démédullé

Des rats mâles Sprague-Dawley (250 à 350 g) sont anesthésiés par une injection intra-péritonéale de pentobarbital sodique (50 mg/kg). La pression artérielle diastolique est enregistrée grâce à un cathéter (PE50) hépariné introduit dans la carotide gauche de l'animal, et relié à un calculateur de pression (Gould, Pressure Processor) par l'intermédiaire d'un capteur de pression Gould.

Un cathéter est introduit dans la jugulaire droite de l'animal afin de permettre l'injection des molécules à étudier.

L'animal est placé sous respiration assistée. Une section bilatérale des nerfs vagues est effectuée. Le rat est alors démédullé.

Après une période de stabilisation suffisante, l'étude de l'antagonisme des molécules vis-à-vis de l'angiotensine II (Hypertensine, CIBA) est abordée de la façon suivante :
1 - Trois injections consécutives d'angiotensine II (0,75 microgrammes/kg) espacées de 15 minutes permettent d'obtenir une réponse pressive reproductible et stable.
2 - Tout en gardant une périodicité de 15 minutes pour l'administration d'angiotensine II, les molécules (0,01 à 10 mg/kg) sont injectées 5 minutes avant l'angiotensine II.
Les effets presseurs de l'angiotensine II en présence de l'antagoniste sont exprimés en pourcentage des effets presseurs de l'angiotensine II administrée seule. La dose inhibitrice à 50 % de l'effet étudié est ainsi déterminée (DI₅₀).
Chaque animal est considéré comme son propre témoin.

| Résultats : | |
|---|---|
| Produit de l'exemple | DI₅₀ en mg/kg |
| 4 | 0,3 |

## Revendications (Revendications pour l'(les) Etat(s) contractant(s) suivant(s): AT, BE, CH, DE, DK, FR, GB, IT, LI, LU, NL, SE)

1. Produits de formule (I_{B}) : 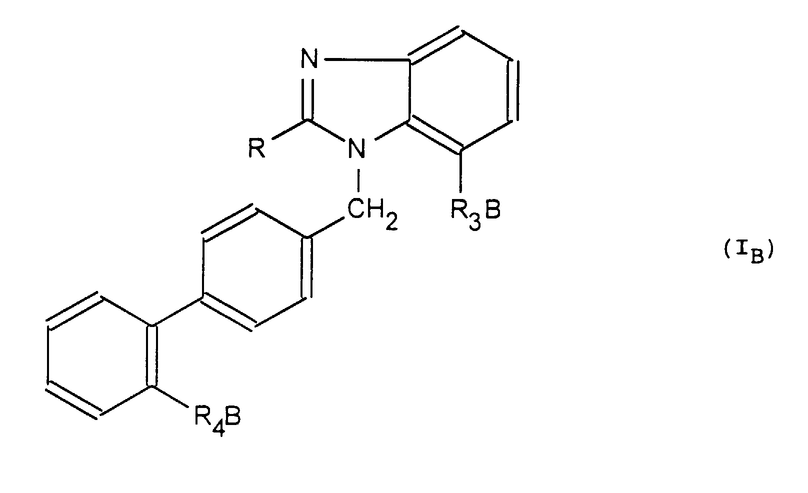 dans laquelle :
R représente un radical butyle ;
R_{3B} représente un radical -COOR₇ dans lequel R₇ représente un atome d'hydrogène ou un radical alkyle ou alkényle linéaire ou ramifié renfermant au plus 5 atomes de carbone ;
R_{4B} représente un radical carboxy libre, estérifié ou salifié; lesdits produits de formule (I_{B}) étant sous toutes les formes isomères possibles racémiques, énantiomères et diastéréoisomères, ainsi que les sels d'addition avec les acides minéraux ou organiques et les bases minérales ou organiques desdits produits de formule (I_{B}).

2. Produits de formule (I_{B}) selon la revendication 1, dans laquelle R_{3B} est COOH.

3. Produits de formule (I_{B}) selon la revendication 1 ou 2, répondant à la formule :
acide 2-butyl 1-[2'-carboxy biphényl-4-yl) méthyl] 1H-benzimidazole-7-carboxylique et ses sels.

4. Procédé de préparation de produits de formule (I_{B}) telle que définie à la revendication 1 caractérisé en ce que : **soit** on fait réagir un produit de formule (IV_{B}) : 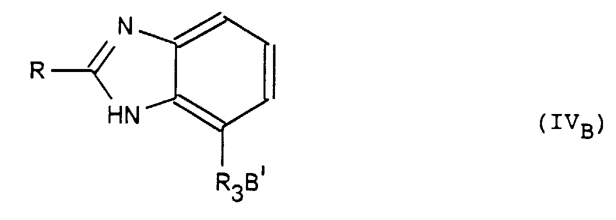 dans laquelle R et R_{3B}' ont les significations indiquées à la revendication 1, respectivement pour R et R_{3B} dans lesquelles les éventuelles fonctions réactives sont éventuellement protégées par des groupements protecteurs, avec un composé de formule (V_{B}) : 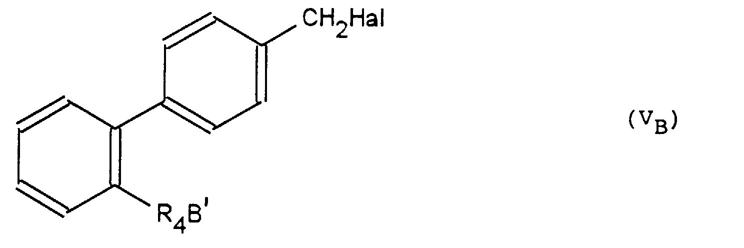 dans laquelle Hal représente un atome d'halogène et R_{4B}' a la signification indiquée à la revendication 1 pour R_{4B} dans lequel les éventuelles fonctions réactives sont éventuellement protégées par des groupements protecteurs, pour obtenir des produits de formule (IX_{B}) : 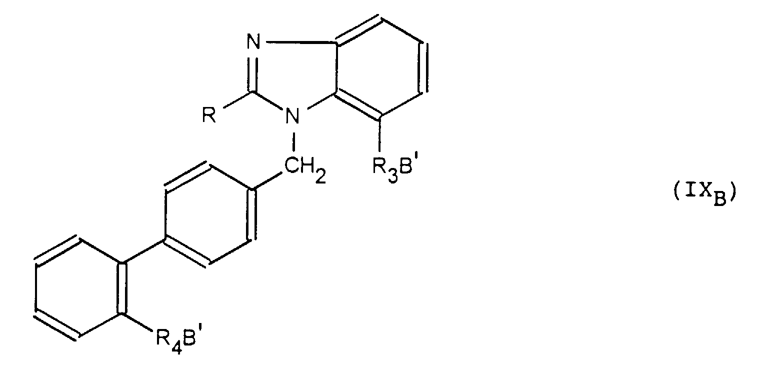 dans laquelle R, R_{3B}' et R_{4B}' ont les significations indiquées ci-dessus,
**soit** a) l'on fait réagir un composé de formule (VI_{B}) : 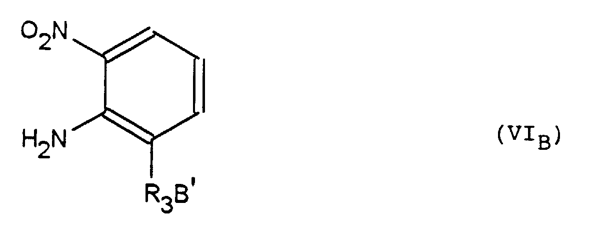 dans laquelle R_{3B}' a la signification précédente, ou bien avec le composé de formule (II) : 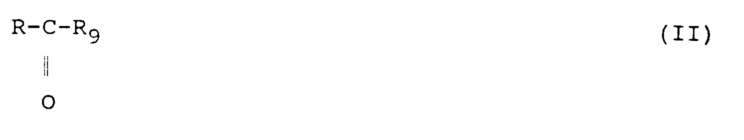 dans laquelle R₉ représente un radical hydroxy, alkyloxy ou un atome d'halogène et R a la signification indiquée ci-dessus, pour obtenir le produit de formule (X_{B}) : 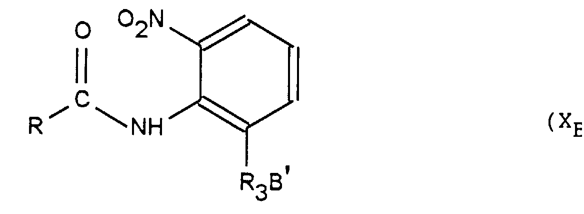 dans laquelle R et R_{3B}' ont les significations précédentes,
b) l'on fait réagir un composé de formule (VI_{B}') : 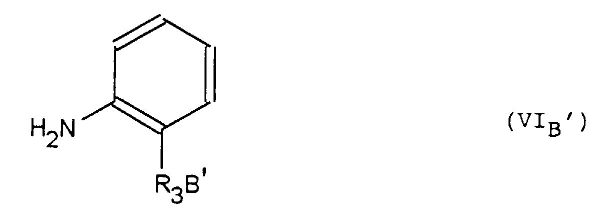 dans laquelle R_{3B}' a la signification précédente, avec le composé de formule (II) tel que défini ci-dessus pour obtenir le produit de formule (X_{B}') : 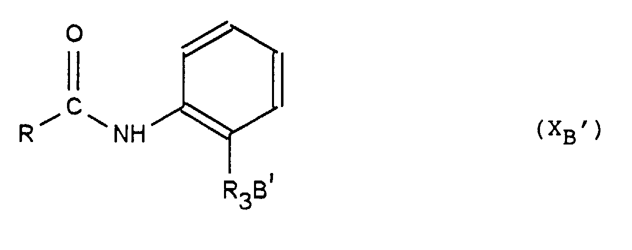 dans laquelle R_{3B}' et R ont les significations indiquées précédemment,
que l'on soumet à une réaction de nitration pour obtenir le composé de formule (X_{B}) tel que défini ci-dessus, produit de formule (X_{B}) que l'on fait réagir avec le composé de formule (V_{B}) telle que définie ci-dessus, pour obtenir un produit de formule (XI_{B}) : 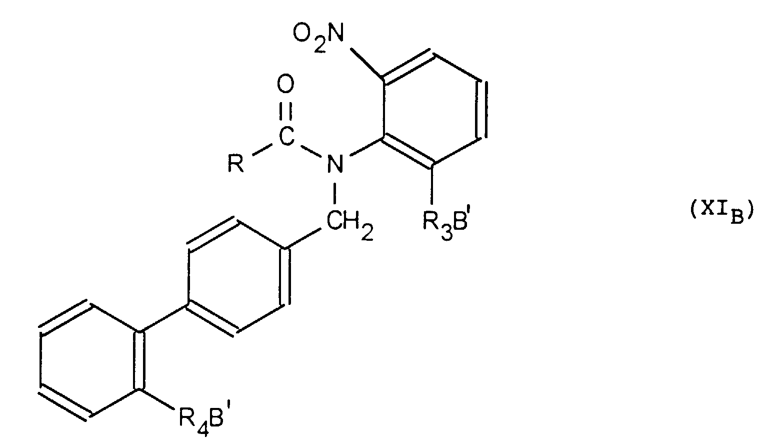 dans laquelle R, R_{3B}' et R_{4B}' ont les significations précédentes, que l'on soumet à une réaction de réduction sélective de la fonction nitro, pour obtenir le produit de formule (XII_{B}) : 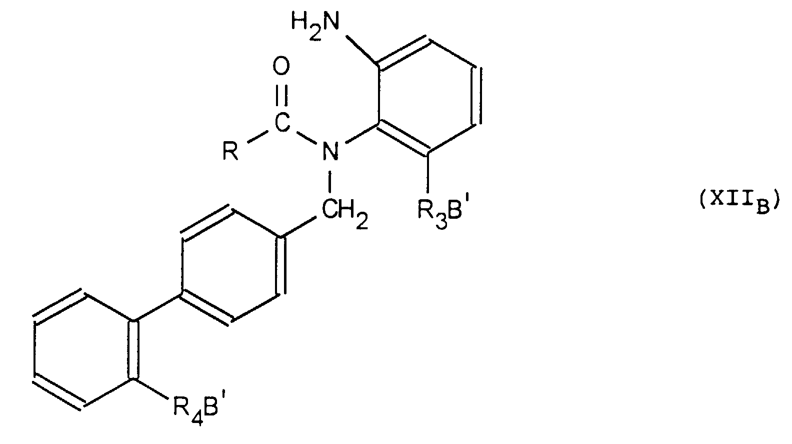 dans laquelle R, R_{3B}' et R_{4B}' ont les significations précédentes, que l'on soumet à une réaction de cyclisation pour obtenir des produits de formule (IX_{B}) telle que définie ci-dessus,
**soit** l'on fait réagir le composé de formule (VI_{B}) avec le composé de formule (V_{B}) telle que définie ci-dessus, pour obtenir des produits de formule (VII_{B}) : 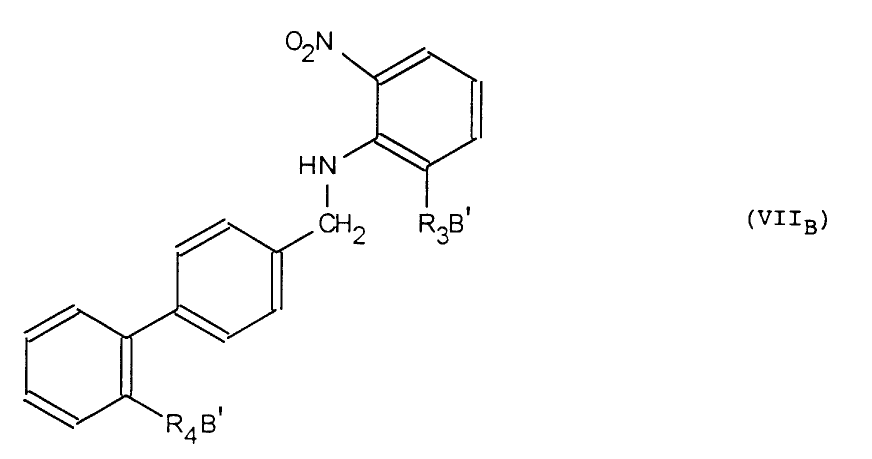 dans laquelle R_{3B}' et R_{4B}' ont les significations indiquées ci-dessus, que l'on soumet à une réaction de réduction du radical nitro en radical amino pour obtenir des produits de formule (VIII_{B}) : 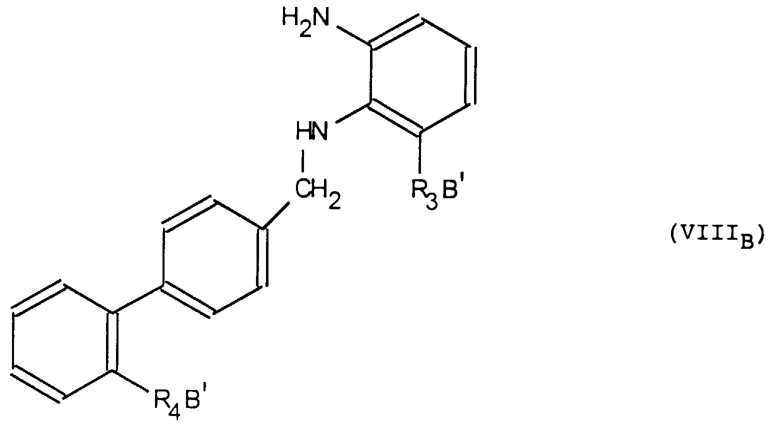 dans laquelle R_{3B}' et R_{4B}' ont les significations indiquées ci-dessus, que l'on fait réagir avec le produit de formule (III) : 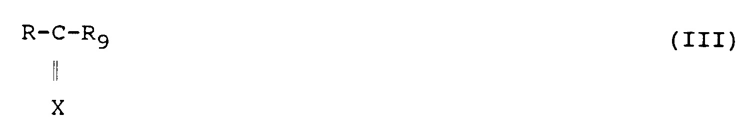 dans laquelle X représente un atome d'oxygène ou un radical NH, R₉ et R ont la signification indiquée ci-dessus, pour obtenir après cyclisation, un produit de formule (IX_{B}) telle que définie ci-dessus,
produit de formule (IX_{B}) que l'on soumet, si désiré et si nécessaire, à l'une ou plusieurs des réactions suivantes, dans un ordre quelconque :
- une réaction d'élimination des groupements protecteurs que peuvent porter les fonctions réactives protégées,
- une réaction de salification par un acide ou une base minéral ou organique pour obtenir le sel correspondant,
- une réaction d'estérification ou salification d'une fonction acide,
- une réaction d'hydrolyse acide ou alcaline d'une fonction ester en fonction acide,
- une réaction de dédoublement des formes racémiques en produits dédoublés,
lesdits produits de formule (I_{B}) ainsi obtenus étant sous toutes les formes isomères possibles racémiques, énantiomères et diastéréoisomères.

5. A titre de médicaments, les produits de formule (I_{B}) tels que définis aux revendications 1 à 3, lesdits produits de formule (I_{B}) étant sous toutes les formes isomères possibles racémiques, énantiomères et diastéréoisomères, ainsi que les sels d'addition avec les acides minéraux ou organiques et les bases minérales ou organiques pharmaceutiquement acceptables desdits produits de formule (I_{B}).

6. Les compositions pharmaceutiques contenant à titre de principe actif, l'un au moins des médicaments tels que définis à la revendication 5.

7. Les composés de formules (VII_{B}) et (VIII_{B}) telles définies à la revendication 4.

8. Utilisation des produits selon l'une des revendications 1 à 3 pour la préparation d'un médicament destiné au traitement de l'hypertension artérielle, des insuffisances cardiaques, des insuffisances rénales, à la prévention des resténoses post-angioplastie, au traitement de désordres gastro-intestinaux, gynécologiques pour leur effet relaxant au niveau de l'utérus.

## Revendications (Revendications pour l'(les) Etat(s) contractant(s) suivant(s): ES)

1. Procédé pour préparer des produits de formule (I_{B}) : 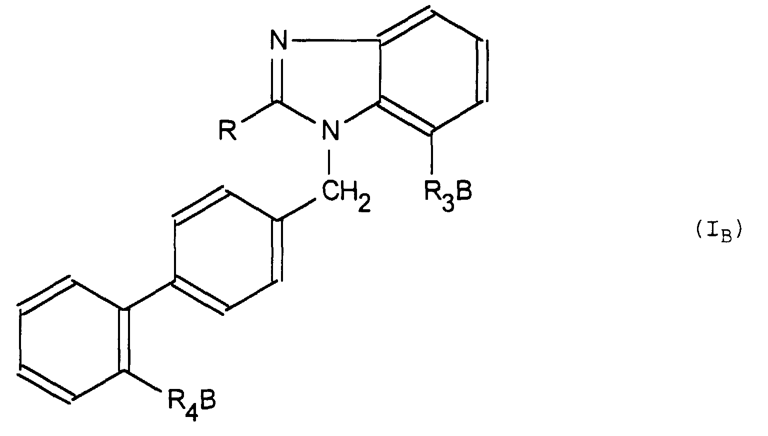 dans laquelle :
R représente un radical butyle ;
R_{3B} représente un radical -COOR₇ dans lequel R₇ représente un atome d'hydrogène ou un radical alkyle ou alkényle linéaire ou ramifié renfermant au plus 5 atomes de carbone ;
R_{4B} représente un radical carboxy libre, estérifié ou salifié; lesdits produits de formule (I_{B}) étant sous toutes les formes isomères possibles racémiques, énantiomères et diastéréoisomères, ainsi que les sels d'addition avec les acides minéraux ou organiques et les bases minérales ou organiques desdits produits de formule (I_{B}), caractérisé en ce que
**soit** on fait réagir un produit de formule (IV_{B}) : 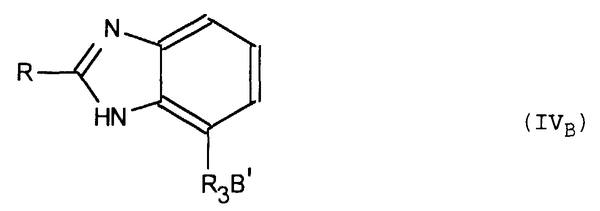 dans laquelle R et R_{3B}' ont les significations indiquées ci-dessus, respectivement pour R et R_{3B} dans lesquelles les éventuelles fonctions réactives sont éventuellement protégées par des groupements protecteurs, avec un composé de formule (V_{B}) : 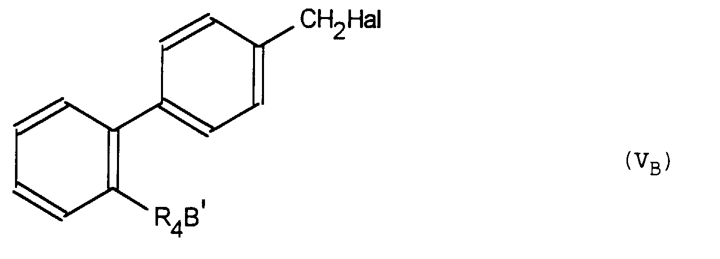 dans laquelle Hal représente un atome d'halogène et R_{4B}' a la signification indiquée ci-dessus pour R_{4B} dans lequel les éventuelles fonctions réactives sont éventuellement protégées par des groupements protecteurs, pour obtenir des produits de formule (IX_{B}) : 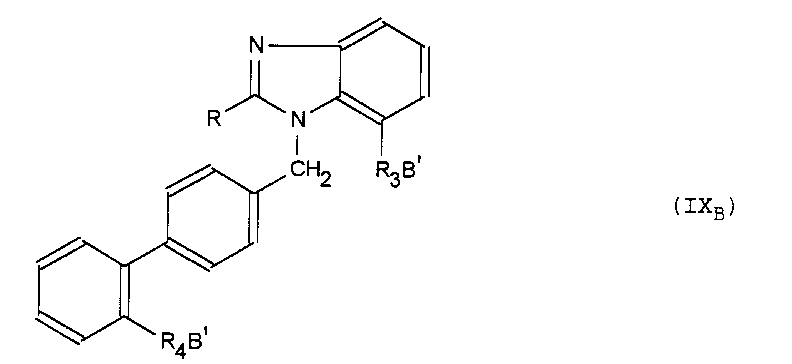 dans laquelle R, R_{3B}' et R_{4B}' ont les significations indiquées ci-dessus,
**soit** a) l'on fait réagir un composé de formule (VI_{B}) : 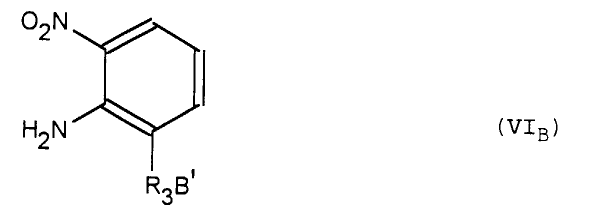 dans laquelle R_{3B}' a la signification précédente, ou bien avec le composé de formule (II) : 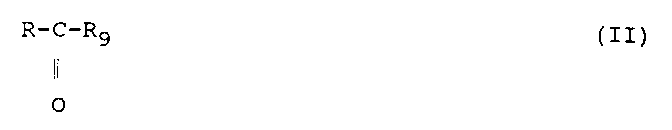 dans laquelle R₉ représente un radical hydroxy, alkyloxy ou un atome d'halogène et R a la signification indiquée ci-dessus, pour obtenir le produit de formule (X_{B}) : 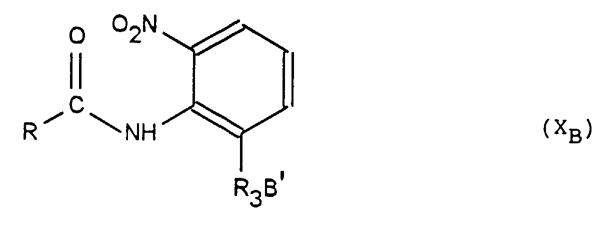 dans laquelle R et R_{3B}' ont les significations précédentes,
b) l'on fait réagir un composé de formule (VI_{B}') : 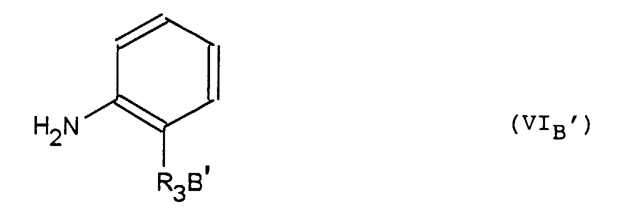 dans laquelle R_{3B}' a la signification précédente, avec le composé de formule (II) tel que défini ci-dessus pour obtenir le produit de formule (X_{B}') : 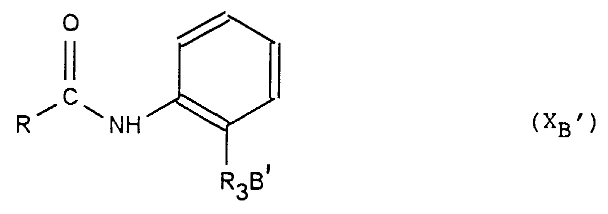 dans laquelle R_{3B}' et R ont les significations indiquées précédemment,
que l'on soumet à une réaction de nitration pour obtenir le composé de formule (X_{B}) tel que défini ci-dessus,
produit de formule (X_{B}) que l'on fait réagir avec le composé de formule (V_{B}) telle que définie ci-dessus, pour obtenir un produit de formule (XI_{B}) : 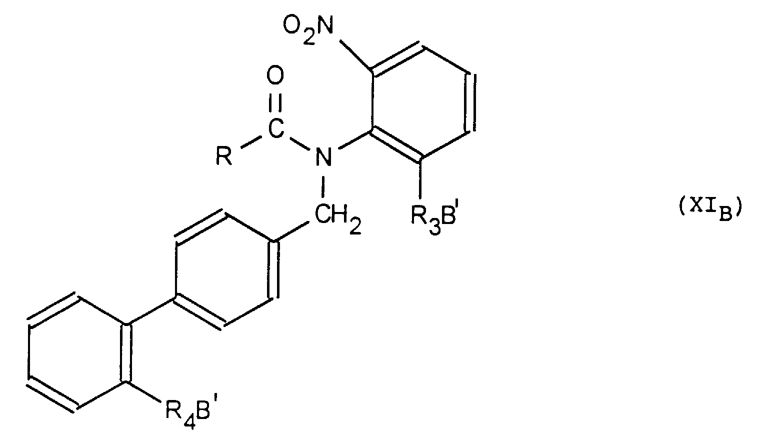 dans laquelle R, R_{3B}' et R_{4B}' ont les significations précédentes, que l'on soumet à une réaction de réduction sélective de la fonction nitro, pour obtenir le produit de formule (XII_{B}) : 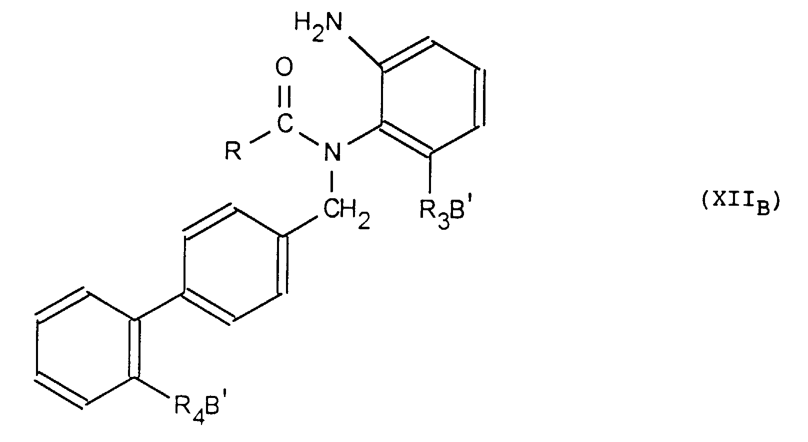 dans laquelle R, R_{3B}' et R_{4B}' ont les significations précédentes, que l'on soumet à une réaction de cyclisation pour obtenir des produits de formule (IX_{B}) telle que définie ci-dessus, **soit** l'on fait réagir le composé de formule (VI_{B}) avec le composé de formule (V_{B}) telle que définie ci-dessus, pour obtenir des produits de formule (VII_{B}) : 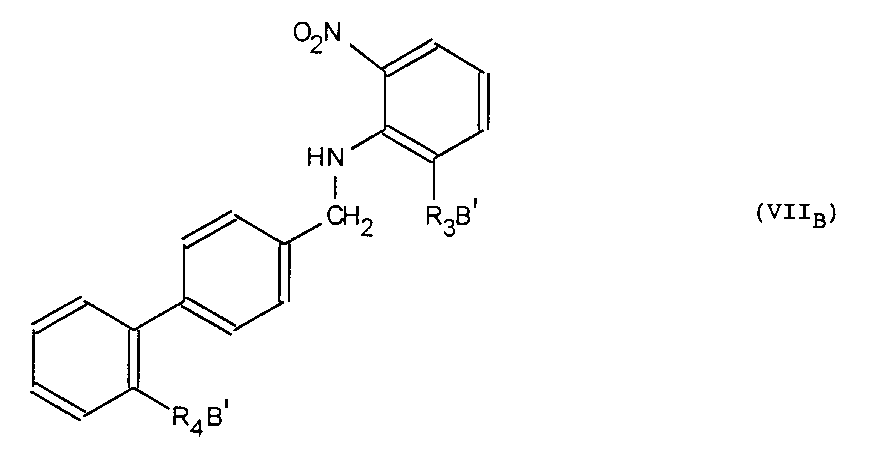 dans laquelle R_{3B}' et R_{4B}' ont les significations indiquées ci-dessus, que l'on soumet à une réaction de réduction du radical nitro en radical amino pour obtenir des produits de formule (VIII_{B}) : 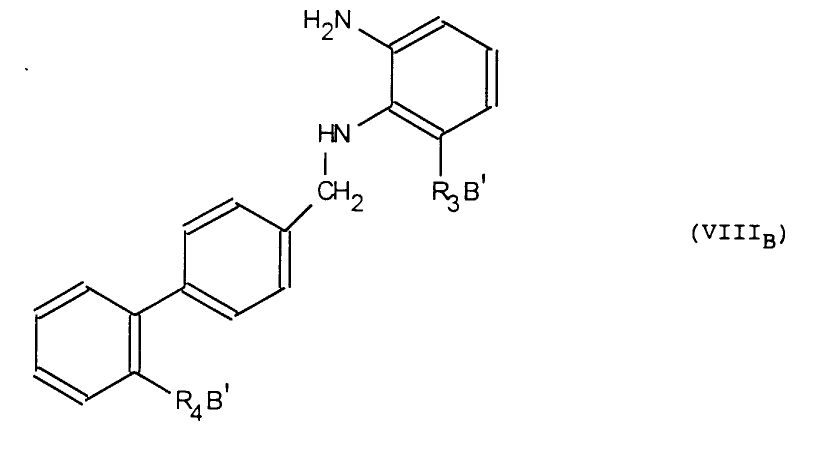 dans laquelle R_{3B}' et R_{4B}' ont les significations indiquées ci-dessus, que l'on fait réagir avec le produit de formule (III) : 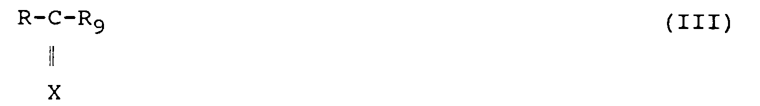 dans laquelle X représente un atome d'oxygène ou un radical NH, R₉ et R ont la signification indiquée ci-dessus, pour obtenir après cyclisation, un produit de formule (IX_{B}) telle que définie ci-dessus,
produit de formule (IX_{B}) que l'on soumet, si désiré et si nécessaire, à l'une ou plusieurs des réactions suivantes, dans un ordre quelconque :
- une réaction d'élimination des groupements protecteurs que peuvent porter les fonctions réactives protégées,
- une réaction de salification par un acide ou une base minéral ou organique pour obtenir le sel correspondant,
- une réaction d'estérification ou salification d'une fonction acide,
- une réaction d'hydrolyse acide ou alcaline d'une fonction ester en fonction acide,
- une réaction de dédoublement des formes racémiques en produits dédoublés,
lesdits produits de formule (I_{B}) ainsi obtenus étant sous toutes les formes isomères possibles racémiques, énantiomères et diastéréoisomères.

## Revendications (Revendications pour l'(les) Etat(s) contractant(s) suivant(s): GR)

1. Procédé pour préparer des produits de formule (I_{B}) : 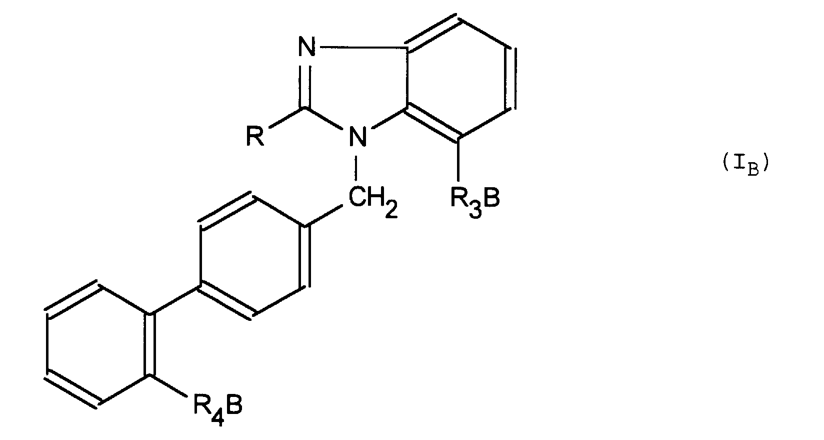 dans laquelle :
R représente un radical butyle ;
R_{3B} représente un radical -COOR₇ dans lequel R₇ représente un atome d'hydrogène ou un radical alkyle ou alkényle linéaire ou ramifié renfermant au plus 5 atomes de carbone ;
R_{4B} représente un radical carboxy libre, estérifié ou salifié; lesdits produits de formule (I_{B}) étant sous toutes les formes isomères possibles racémiques, énantiomères et diastéréoisomères, ainsi que les sels d'addition avec les acides minéraux ou organiques et les bases minérales ou organiques desdits produits de formule (I_{B}), caractérisé en ce que
**soit** on fait réagir un produit de formule (IV_{B}) : 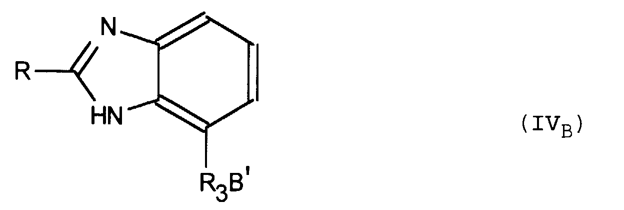 dans laquelle R et R_{3B}' ont les significations indiquées ci-dessus, respectivement pour R et R_{3B} dans lesquelles les éventuelles fonctions réactives sont éventuellement protégées par des groupements protecteurs, avec un composé de formule (V_{B}) : 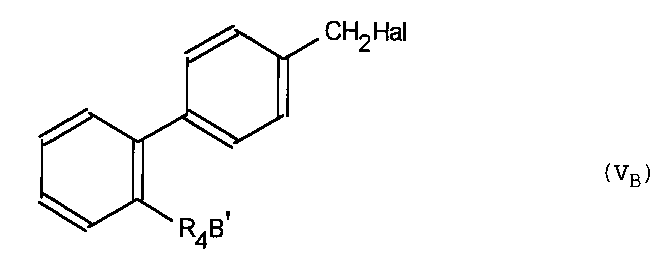 dans laquelle Hal représente un atome d'halogène et R_{4B}' a la signification indiquée ci-dessus pour R_{4B} dans lequel les éventuelles fonctions réactives sont éventuellement protégées par des groupements protecteurs, pour obtenir des produits de formule (IX_{B}) : 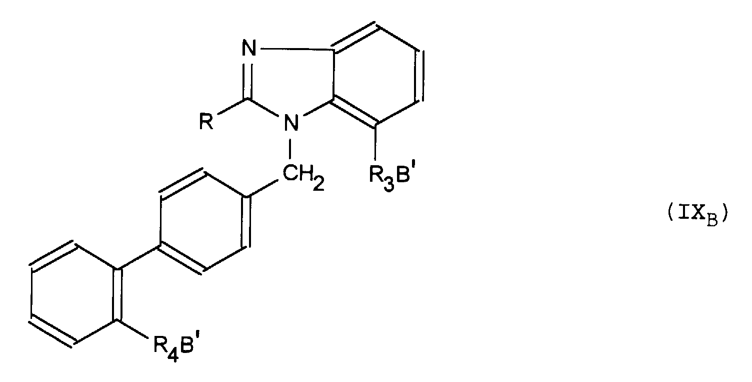 dans laquelle R, R_{3B}' et R_{4B}' ont les significations indiquées ci-dessus,
**soit** a) l'on fait réagir un composé de formule (VI_{B}) : 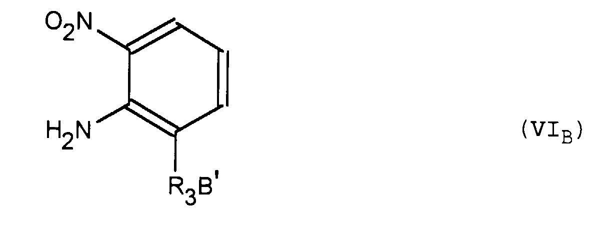 dans laquelle R_{3B}' a la signification précédente, ou bien avec le composé de formule (II) : 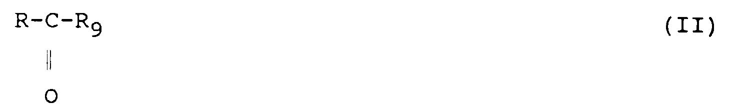 dans laquelle R₉ représente un radical hydroxy, alkyloxy ou un atome d'halogène et R a la signification indiquée ci-dessus, pour obtenir le produit de formule (X_{B}) : 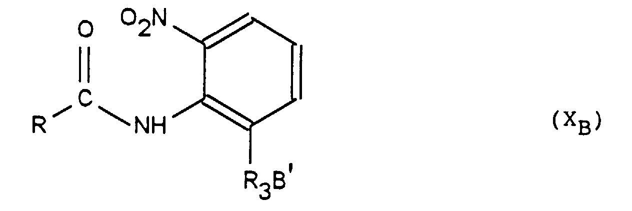 dans laquelle R et R_{3B}' ont les significations précédentes,
b) l'on fait réagir un composé de formule (VI_{B}') : 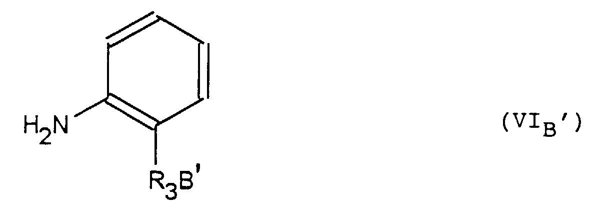 dans laquelle R_{3B}' a la signification précédente, avec le composé de formule (II) tel que défini ci-dessus pour obtenir le produit de formule (X_{B}') : 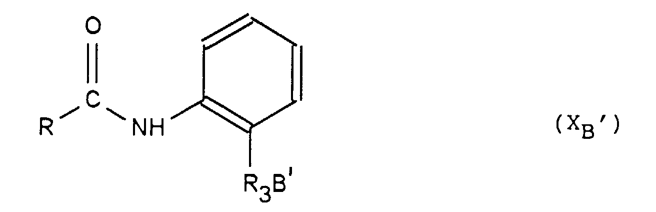 dans laquelle R_{3B}' et R ont les significations indiquées précédemment,
que l'on soumet à une réaction de nitration pour obtenir le composé de formule (X_{B}) tel que défini ci-dessus,
produit de formule (X_{B}) que l'on fait réagir avec le composé de formule (V_{B}) telle que définie ci-dessus, pour obtenir un produit de formule (XI_{B}) : 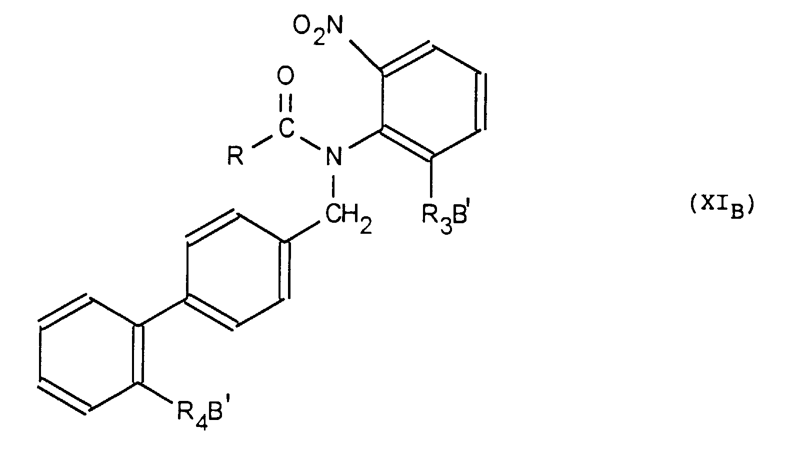 dans laquelle R, R_{3B}' et R_{4B}', ont les significations précédentes, que l'on soumet à une réaction de réduction sélective de la fonction nitro, pour obtenir le produit de formule (XII_{B}) : 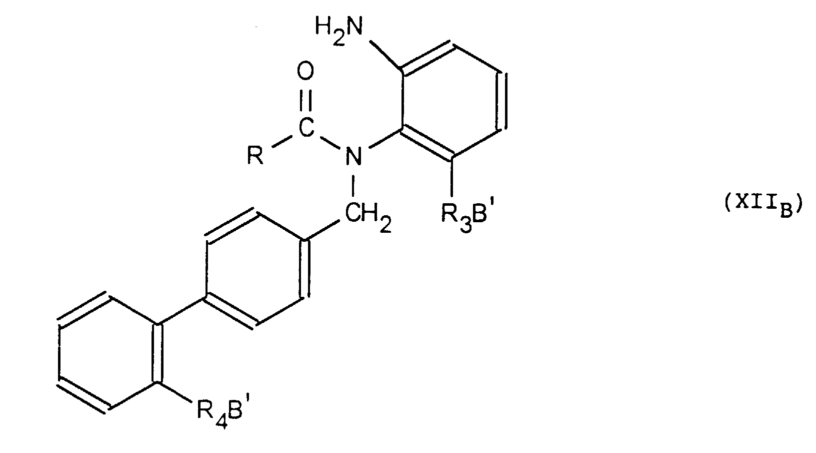 dans laquelle R, R_{3B}' et R_{4B}' ont les significations précédentes, que l'on soumet à une réaction de cyclisation pour obtenir des produits de formule (IX_{B}) telle que définie ci-dessus, **soit** l'on fait réagir le composé de formule (VI_{B}) avec le composé de formule (V_{B}) telle que définie ci-dessus, pour obtenir des produits de formule (VII_{B}) : 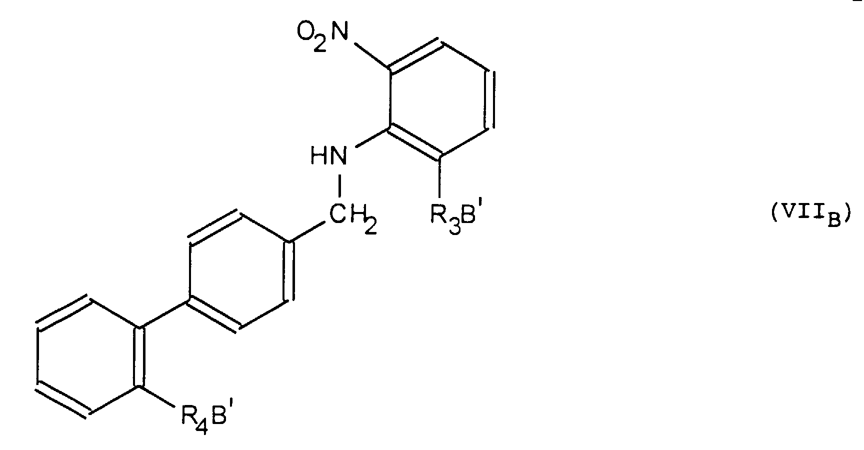 dans laquelle R_{3B}' et R_{4B}' ont les significations indiquées ci-dessus, que l'on soumet à une réaction de réduction du radical nitro en radical amino pour obtenir des produits de formule (VIII_{B}) : 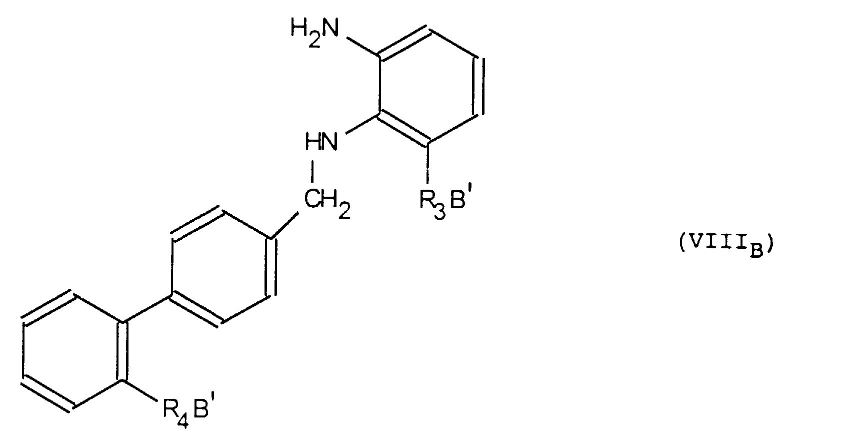 dans laquelle R_{3B}' et R_{4B}' ont les significations indiquées ci-dessus, que l'on fait réagir avec le produit de formule (III) : 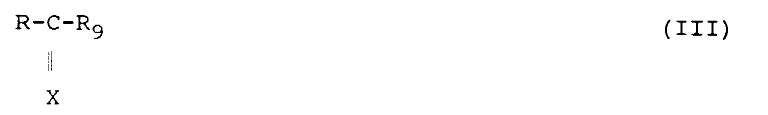 dans laquelle X représente un atome d'oxygène ou un radical NH, R₉ et R ont la signification indiquée ci-dessus, pour obtenir après cyclisation, un produit de formule (IX_{B}) telle que définie ci-dessus,
produit de formule (IX_{B}) que l'on soumet, si désiré et si nécessaire, à l'une ou plusieurs des réactions suivantes, dans un ordre quelconque :
- une réaction d'élimination des groupements protecteurs que peuvent porter les fonctions réactives protégées,
- une réaction de salification par un acide ou une base minéral ou organique pour obtenir le sel correspondant,
- une réaction d'estérification ou salification d'une fonction acide,
- une réaction d'hydrolyse acide ou alcaline d'une fonction ester en fonction acide,
- une réaction de dédoublement des formes racémiques en produits dédoublés,
lesdits produits de formule (I_{B}) ainsi obtenus étant sous toutes les formes isomères possibles racémiques, énantiomères et diastéréoisomères.

2. Procédé de préparation de compositions pharmaceutiques caractérisé en ce que l'on met à titre de principe actif l'un au moins des produits de formule (I_{B}) : 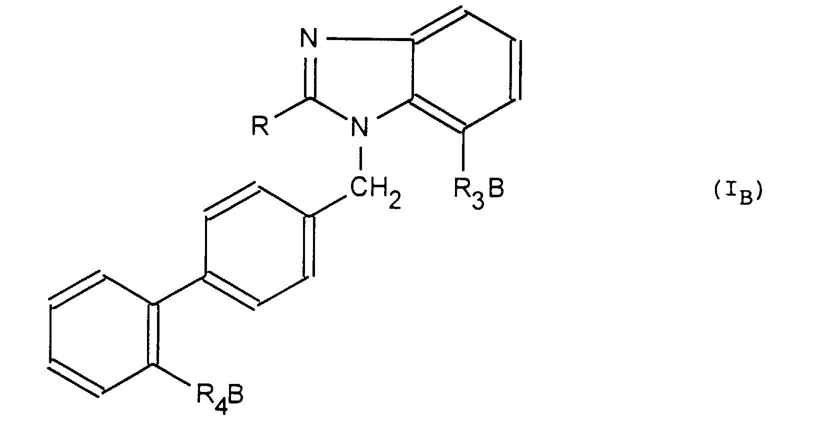 dans laquelle :
R représente un radical butyle ;
R_{3B} représente un radical -COOR₇ dans lequel R₇ représente un atome d'hydrogène ou un radical alkyle ou alkényle linéaire ou ramifié renfermant au plus 5 atomes de carbone ;
R_{4B} représente un radical carboxy libre, estérifié ou salifié ;
lesdits produits de formule (I_{B}) étant sous toutes les formes isomères possibles racémiques, énantiomères et diastéréoisomères, ainsi que les sels d'addition avec les acides minéraux ou organiques et les bases minérales ou organiques desdits produits de formule (I_{B}).

3. Procédé de préparation de compositions pharmaceutiques selon la revendication 2, dans lequel le produit de formule (I_{B}) est tel que R_{3B} est COOH.

4. Procédé de préparation de compositions pharmaceutiques selon la revendication 2, dans lequel le produit de formule (I_{B}) répond à la formule :
acide 2-butyl 1-[2'-carboxy biphényl-4-yl) méthyl] 1H-benzimidazole-7-carboxylique et ses sels.

## Patentansprüche (Patentansprüche für folgende(n) Vertragsstaat(en): AT, BE, CH, DE, DK, FR, GB, IT, LI, LU, NL, SE)

1. Produkte der Formel (I_{B}) 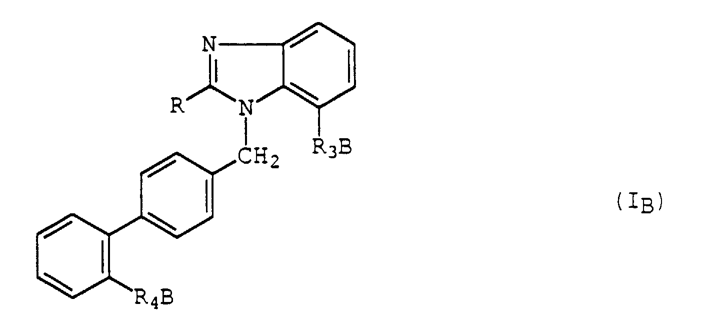 in der
R einen Rest Butyl darstellt;
R₃B einen Rest -COOR₇ bedeutet, worin R₇ ein Wasserstoffatom oder ein gerader oder verzweigter Rest Alkyl oder Alkenyl mit höchstens 5 Kohlenstoffatomen ist;
R₄B einen freien, veresterten oder in Salz überführten Rest Carboxy darstellt,
wobei die genannten Produkte der Formel (I_{B}) in allen möglichen racemischen, enantiomeren oder diastereoisomeren Isomerie-Formen vorliegen, sowie die Additionssalze der genannten Produkte der Formel (I_{B}) mit Mineralsäuren oder organischen Säuren und Mineralbasen oder organischen Basen.

2. Produkte der Formel (I_{B}) nach Anspruch 1, worin R₃B COOH ist.

3. Produkte der Formel (I_{B}) nach Anspruch 1 oder 2, die der folgenden Formel entsprechen:
2-Butyl-1-[(2'-carboxy-biphenyl-4-yl)-methyl]-1H-benzimidazol-7-carbonsäure und ihre Salze.

4. Verfahren zur Herstellung der Produkte der Formel (I_{B}) wie in Anspruch 1 definiert, dadurch gekennzeichnet, daß man entweder ein Produkt der Formel (IV_{B}) 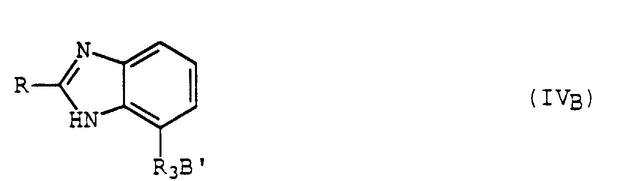 in der R und R₃B' die in Anspruch 1 jeweils für R und R₃B angegebenen Bedeutungen besitzen, bei denen die eventuellen reaktiven Funktionen gegebenenfalls durch Schutzgruppen geschützt sind, mit einer Verbindung der Formel (V_{B}) 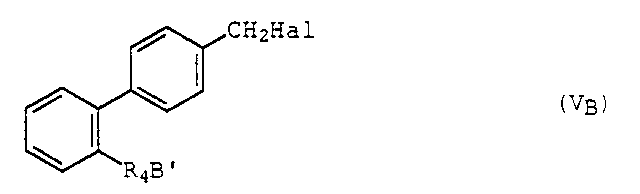 zur Reaktion bringt, in der Hal ein Halogenatom darstellt und R₄B' die in Anspruch 1 für R₄B angegebene Bedeutung besitzt, bei der die eventuellen reaktiven Funktionen gegebenenfalls durch Schutzgruppen geschützt sind, um Produkte der Formel (IX_{B}) 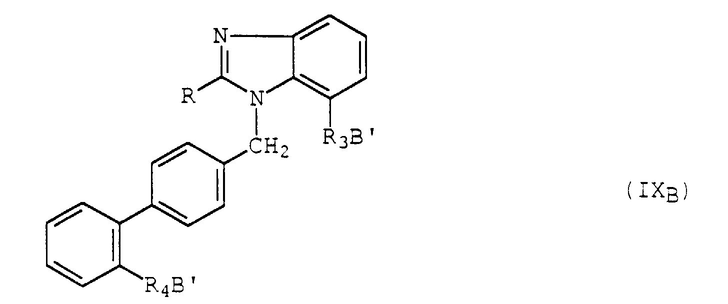 zu erhalten, in der R, R₃B' und R₄B' die oben angegebenen Bedeutungen besitzen,
oder a) eine Verbindung der Formel (VI_{B}) 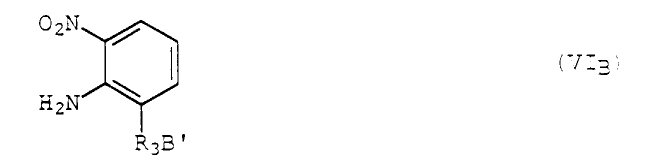 in der R₃B' die obige Bedeutung besitzt, mit der Verbindung der Formel (II) 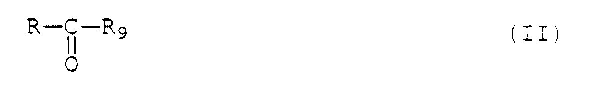 zur Reaktion bringt, in der R₉ einen Rest Hydroxy, Alkoxy oder ein Halogenatom darstellt und R die oben angegebene Bedeutung besitzt, um das Produkt der Formel (X_{B}) 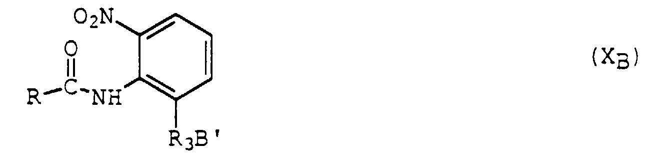 zu erhalten, in der R und R₃B' die vorstehenden Bedeutungen besitzen,
b) eine Verbindung der Formel (VI_{B}') 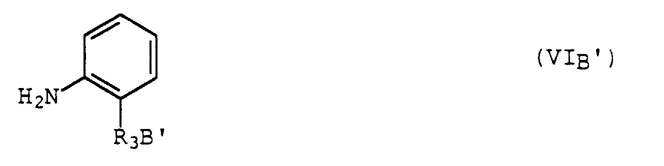 in der R₃B' die vorstehend angegebene Bedeutung besitzt, mit der wie oben definierten Verbindung der Formel (II) zu Reaktion bringt, um das Produkt der Formel (X_{B}') 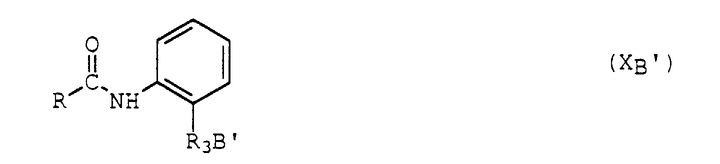 zu erhalten, in der R₃B' und R die oben angegebenen Bedeutungen besitzen,
das man einer Nitrierungs-Reaktion unterzieht, um die wie oben definierte Verbindung der Formel (X_{B}) zu erhalten, und man dieses Produkt Formel (X_{B}) mit der wie oben definierten Verbindung der Formel (V_{B}) zur Reaktion bringt, um ein Produkt der Formel (XI_{B}) 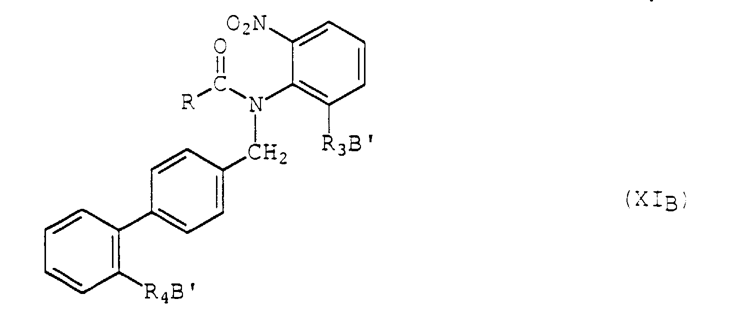 zu erhalten, in der R, R₃B' und R₄B' die vorstehenden Bedeutungen besitzen, das man einer Reaktion zur selektiven Reduktion der Nitrofunktion unterzieht, um das Produkt der Formel (XII_{B}) 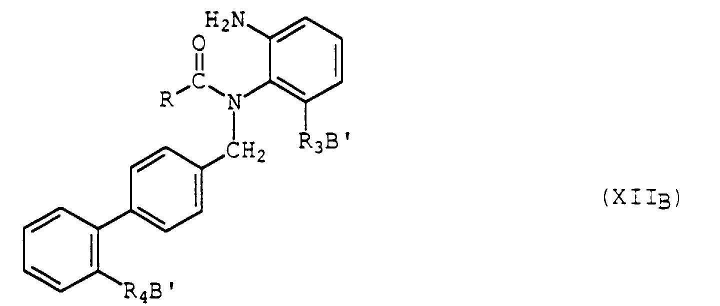 zu erhalten, in der R, R₃B' und R₄B' die vorstehenden Bedeutungen besitzen, das man einer Reaktion zur Cyclisierung unterzieht, um die wie oben definierten Produkte der Formel (IX_{B}) zu erhalten,
oder
die Verbindung der Formel (VI_{B}) mit der wie oben definierten Verbindung der Formel (V_{B}) zur Reaktion bringt, um Produkte der Formel (VII_{B}) 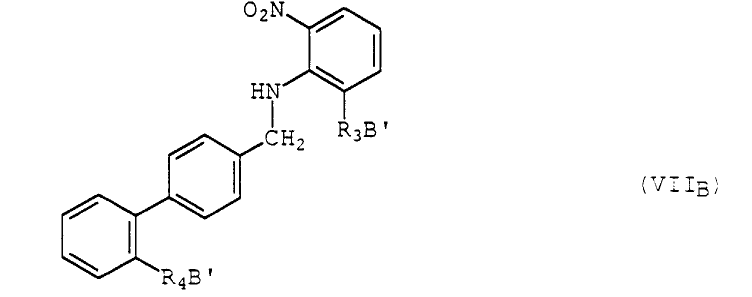 zu erhalten, in der R₃B' und R₄B' die oben angegebenen Bedeutungen besitzen, die man einer Reaktion zur Reduktion des Restes Nitro in einen Rest Amino unterzieht, um die Produkte der Formel (VIII_{B}) 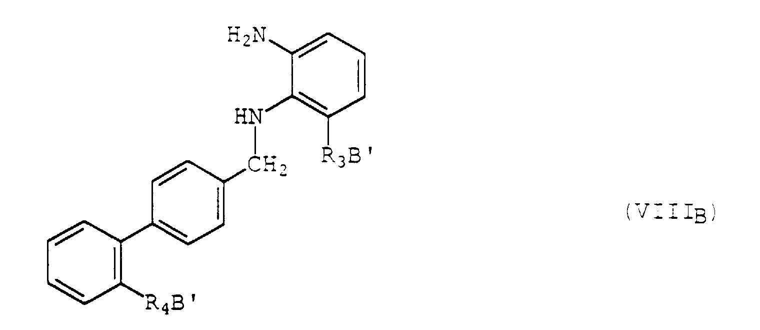 zu erhalten, in der R₃B' und R₄B' die oben angegebenen Bedeutungen besitzen, die man mit dem Produkt der Formel (III) 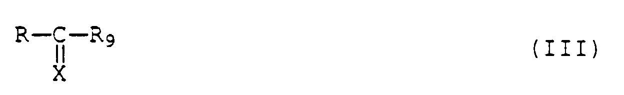 zur Reaktion bringt, in der X ein Sauerstoffatom oder ein Rest NH ist, R₉ und R die oben angegebenen Bedeutungen besitzen, um nach der Cyclisierung ein wie oben definiertes Produkt der Formel (IX_{B}) zu erhalten,
das man, wenn erwünscht und wenn erforderlich, einer oder mehreren der folgenden Reaktionen in irgendeiner Reihenfolge unterwirft:
- einer Reaktion zur Entfernung der Schutzgruppen, die die geschützten reaktiven Funktionen tragen können,
- einer Reaktion zur Salzbildung durch eine Mineralsäure oder organische Säure oder Mineralbase oder organische Base, um das entsprechende Salz zu erhalten,
- einer Reaktion zur Veresterung oder Salzbildung einer Säurefunktion,
- einer Reaktion zur sauren oder alkalischen Hydrolyse einer Esterfunktion in die Säurefunktion,
- einer Reaktion zur Aufspaltung der racemischen Formen in die Spaltprodukte,
wobei die auf diese Weise erhaltenen Produkte der Formel (I_{B}) in allen möglichen racemischen, enantiomeren oder diastereoisomeren Isomerie-Formen vorliegen.

5. Als Arzneimittel die wie in den Ansprüchen 1 bis 3 definierten Produkte der Formel (I_{B}), wobei die genannten Produkte der Formel (I_{B}) in allen möglichen racemischen, enantiomeren oder diastereoisomeren Isomerie-Formen vorliegen, sowie die Additionssalze der genannten Produkte der Formel (I_{B}) mit pharmazeutisch akzeptablen Mineralsäuren oder organischen Säuren und Mineralbasen oder organischen Basen.

6. Pharmazeutische Zusammensetzungen, die als Wirkstoff mindestens eines der wie in Anspruch 5 definierten Arzneimittel enthalten.

7. Verbindungen der Formeln (VII_{B}) und (VIII_{B}) wie in Anspruch 4 definiert.

8. Verwendung der Produkte nach einem der Ansprüche 1 bis 3 zur Herstellung eines Arzneimittels zur Behandlung von arteriellem Überdruck, Herzinsuffizienzen, Niereninsuffizienzen, zur Vorbeugung von post-angioplastischen Restenosen, zur Behandlung von gastro-intestinalen Störungen, zur gynäkologischen Behandlung im Hinblick auf ihre relaxierende Wirkung im Bereich des Uterus.

## Patentansprüche (Patentansprüche für folgende(n) Vertragsstaat(en): ES)

1. Verfahren zur Herstellung von Produkten der Formel (I_{B}) 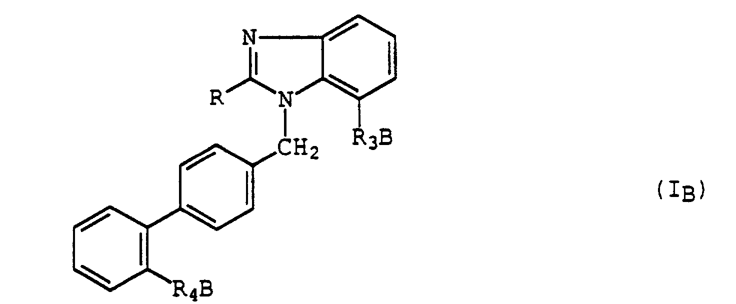 in der
R einen Rest Butyl darstellt;
R₃B einen Rest -COOR₇ bedeutet, worin R₇ ein Wasserstoffatom oder ein gerader oder verzweigter Rest Alkyl oder Alkenyl mit höchstens 5 Kohlenstoffatomen ist;
R₄B einen freien, veresterten oder in Salz überführten Rest Carboxy darstellt,
wobei die genannten Produkte der Formel (I_{B}) in allen möglichen racemischen, enantiomeren oder diastereoisomeren Isomerie-Formen vorliegen, sowie die Additionssalze der genannten Produkte der Formel (I_{B}) mit Mineralsäuren oder organischen Säuren und Mineralbasen oder organischen Basen, dadurch gekennzeichnet, daß man entweder ein Produkt der Formel (IV_{B}) 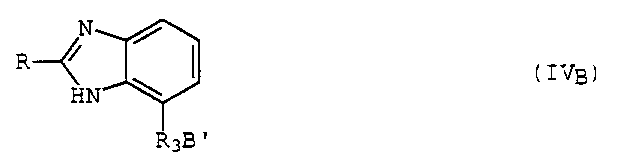 in der R und R₃B' die oben jeweils für R und R₃B angegebenen Bedeutungen besitzen, bei denen die eventuellen reaktiven Funktionen gegebenenfalls durch Schutzgruppen geschützt sind, mit einer Verbindung der Formel (V_{B}) 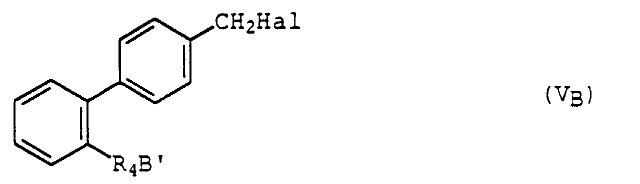 zur Reaktion bringt, in der Hal ein Halogenatom darstellt und R₄B' die oben für R₄B angegebene Bedeutung besitzt, bei der die eventuellen reaktiven Funktionen gegebenenfalls durch Schutzgruppen geschützt sind, um Produkte der Formel (IX_{B}) 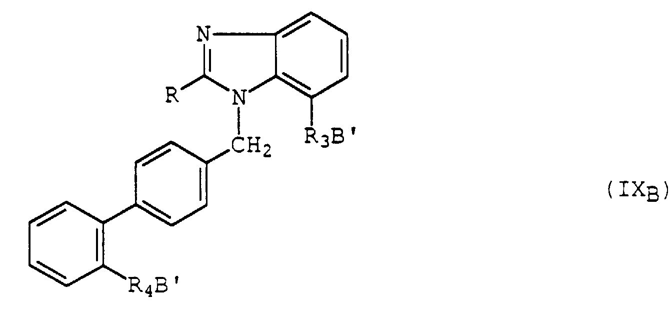 zu erhalten, in der R, R₃B' und R₄B' die oben angegebenen Bedeutungen besitzen,
oder a) eine Verbindung der Formel (VI_{B}) 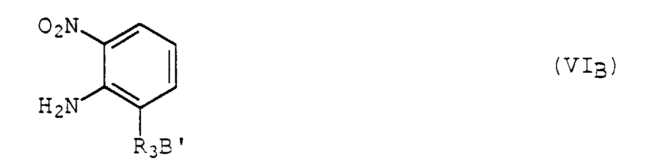 in der R₃B' die obige Bedeutung besitzt, mit der Verbindung der Formel (II) 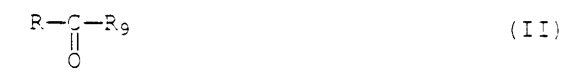 zur Reaktion bringt, in der R₉ einen Rest Hydroxy, Alkoxy oder ein Halogenatom darstellt und R die oben angegebene Bedeutung besitzt, um das Produkt der Formel (X_{B}) 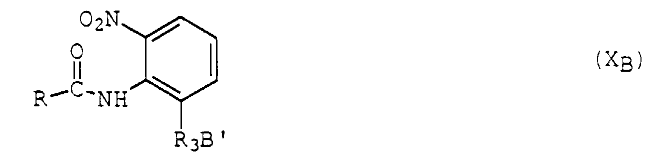 zu erhalten, in der R und R₃B' die vorstehenden Bedeutungen besitzen,
b) eine Verbindung der Formel (VI_{B}') 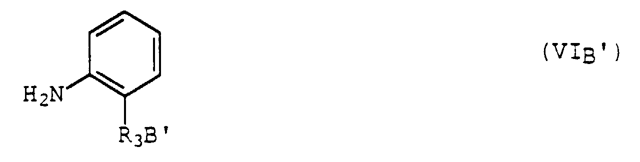 in der R₃B' die vorstehend angegebene Bedeutung besitzt, mit der wie oben definierten Verbindung der Formel (II) zu Reaktion bringt, um das Produkt der Formel (X_{B}') 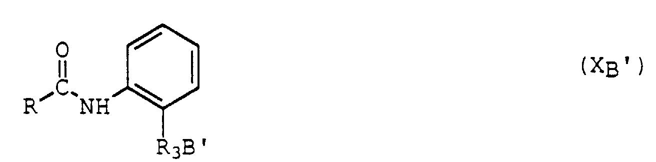 zu erhalten, in der R₃B' und R die oben angegebenen Bedeutungen besitzen,
das man einer Nitrierungs-Reaktion unterzieht, um die wie oben definierte Verbindung der Formel (X_{B}) zu erhalten, und man dieses Produkt Formel (X_{B}) mit der wie oben definierten Verbindung der Formel (V_{B}) zur Reaktion bringt, um ein Produkt der Formel (XI_{B}) 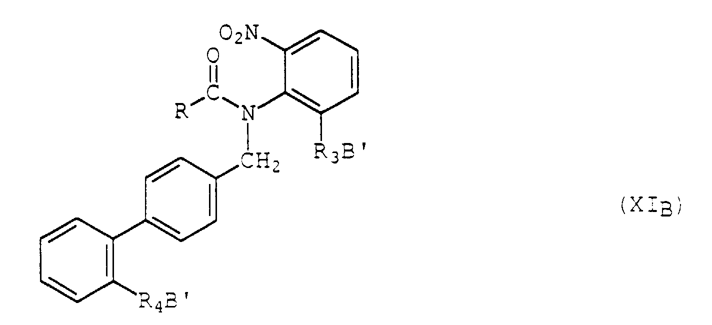 zu erhalten, in der R, R₃B' und R₄B' die vorstehenden Bedeutungen besitzen, das man einer Reaktion zur selektiven Reduktion der Nitrofunktion unterzieht, um das Produkt der Formel (XII_{B}) 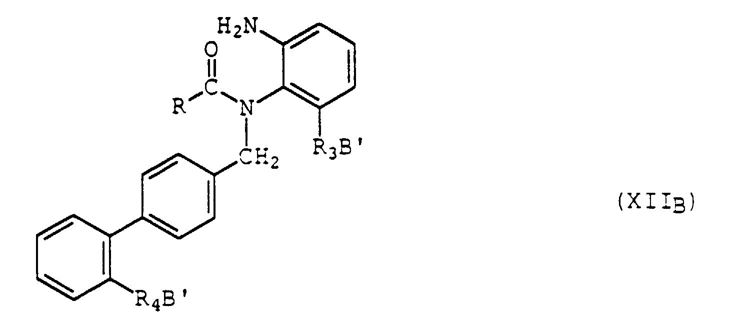 zu erhalten, in der R, R₃B' und R₄B' die vorstehenden Bedeutungen besitzen, das man einer Reaktion zur Cyclisierung unterzieht, um die wie oben definierten Produkte der Formel (IX_{B}) zu erhalten,
oder
die Verbindung der Formel (VI_{B}) mit der wie oben definierten Verbindung der Formel (V_{B}) zur Reaktion bringt, um Produkte der Formel (VII_{B}) 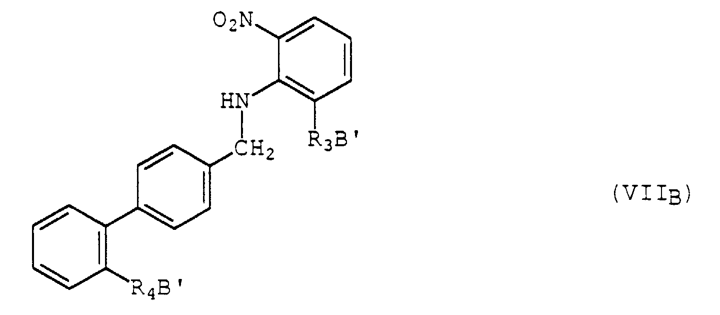 zu erhalten, in der R₃B' und R₄B' die oben angegebenen Bedeutungen besitzen, die man einer Reaktion zur Reduktion des Restes Nitro in einen Rest Amino unterzieht, um die Produkte der Formel (VIII_{B}) 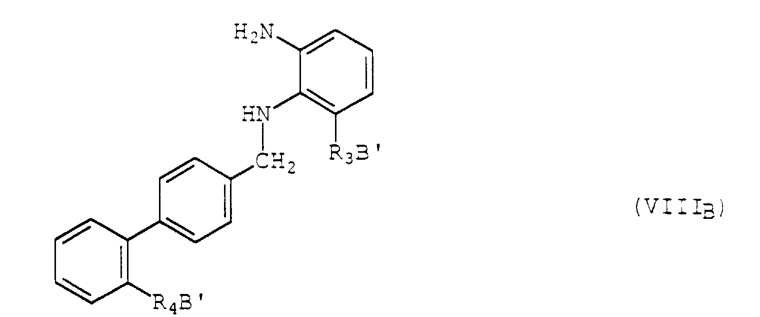 zu erhalten, in der R₃B' und R₄B' die oben angegebenen Bedeutungen besitzen, die man mit dem Produkt der Formel (III) 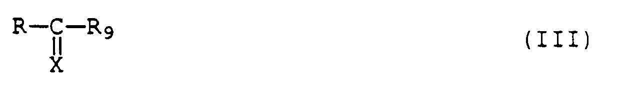 zur Reaktion bringt, in der X ein Sauerstoffatom oder ein Rest NH ist, R₉ und R die oben angegebenen Bedeutungen besitzen, um nach der Cyclisierung ein wie oben definiertes Produkt der Formel (IX_{B}) zu erhalten,
das man, wenn erwünscht und wenn erforderlich, einer oder mehreren der folgenden Reaktionen in irgendeiner Reihenfolge unterwirft:
- einer Reaktion zur Entfernung der Schutzgruppen, die die geschützten reaktiven Funktionen tragen können,
- einer Reaktion zur Salzbildung durch eine Mineralsäure oder organische Säure oder Mineralbase oder organische Base, um das entsprechende Salz zu erhalten,
- einer Reaktion zur Veresterung oder Salzbildung einer Säurefunktion,
- einer Reaktion zur sauren oder alkalischen Hydrolyse einer Esterfunktion in die Säurefunktion,
- einer Reaktion zur Aufspaltung der racemischen Formen in die Spaltprodukte,
wobei die auf diese Weise erhaltenen Produkte der Formel (I_{B}) in allen möglichen racemischen, enantiomeren oder diastereoisomeren Isomerie-Formen vorliegen.

## Patentansprüche (Patentansprüche für folgende(n) Vertragsstaat(en): GR)

1. Verfahren zur Herstellung von Produkten der Formel (I_{B}) 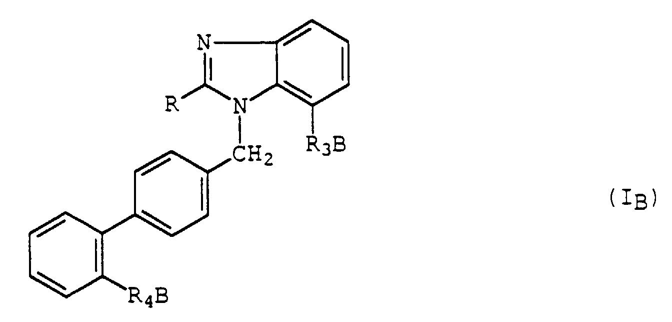 in der
R einen Rest Butyl darstellt;
R₃B einen Rest -COOR₇ bedeutet, worin R₇ ein Wasserstoffatom oder ein gerader oder verzweigter Rest Alkyl oder Alkenyl mit höchstens 5 Kohlenstoffatomen ist;
R₄B einen freien, veresterten oder in Salz überführten Rest Carboxy darstellt,
wobei die Produkte der Formel (I_{B}) in allen möglichen racemischen, enantiomeren oder diastereoisomeren Isomerie-Formen vorliegen, sowie die Additionssalze der genannten Produkte der Formel (I_{B}) mit Mineralsäuren oder organischen Säuren und Mineralbasen oder organischen Basen, dadurch gekennzeichnet, daß man entweder ein Produkt der Formel (IV_{B}) 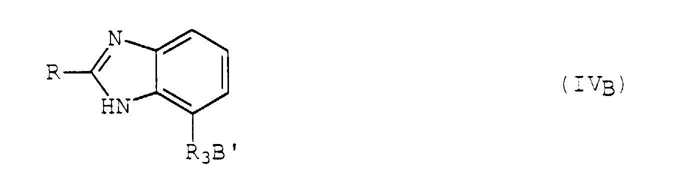 in der R und R₃B' die oben jeweils für R und R₃B angegebenen Bedeutungen besitzen, bei denen die eventuellen reaktiven Funktionen gegebenenfalls durch Schutzgruppen geschützt sind, mit einer Verbindung der Formel (V_{B}) 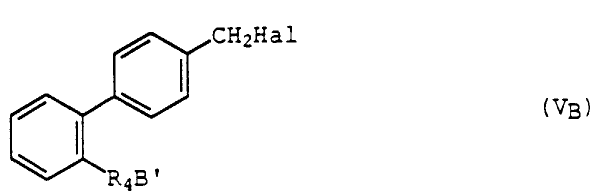 zur Reaktion bringt, in der Hal ein Halogenatom darstellt und R₄B' die oben für R₄B angegebene Bedeutung besitzt, bei der die eventuellen reaktiven Funktionen gegebenenfalls durch Schutzgruppen geschützt sind, um Produkte der Formel (IX_{B}) 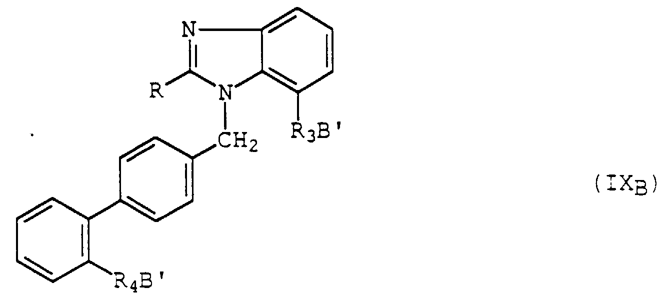 zu erhalten, in der R, R₃B' und R₄B' die oben angegebenen Bedeutungen besitzen,
oder a) eine Verbindung der Formel (VI_{B}) 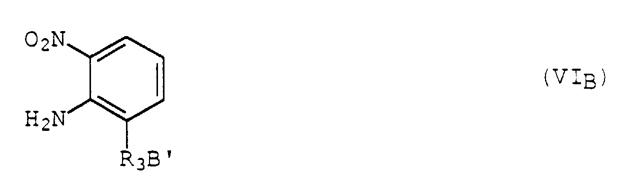 in der R₃B' die obige Bedeutung besitzt, mit der Verbindung der Formel (II) 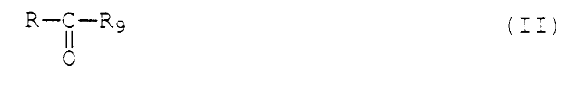 zur Reaktion bringt, in der R₉ einen Rest Hydroxy, Alkoxy oder ein Halogenatom darstellt und R die oben angegebene Bedeutung besitzt, um das Produkt der Formel (X_{B}) 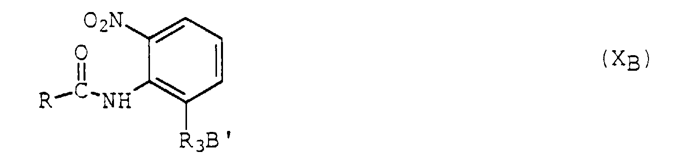 zu erhalten, in der R und R₃B' die vorstehenden Bedeutungen besitzen,
b) eine Verbindung der Formel (VI_{B}') 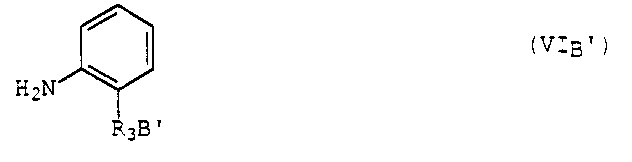 in der R₃B' die vorstehend angegebene Bedeutung besitzt, mit der wie oben definierten Verbindung der Formel (II) zu Reaktion bringt, um das Produkt der Formel (X_{B}') 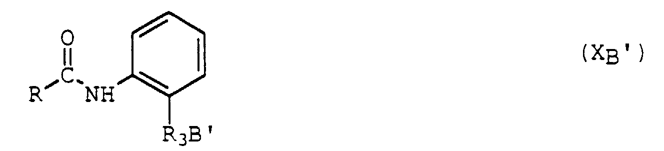 zu erhalten, in der R₃B' und R die oben angegebenen Bedeutungen besitzen,
das man einer Nitrierungs-Reaktion unterzieht, um die wie oben definierte Verbindung der Formel (X_{B}) zu erhalten, und man dieses Produkt Formel (X_{B}) mit der wie oben definierten Verbindung der Formel (V_{B}) zur Reaktion bringt, um ein Produkt der Formel (XI_{B}) 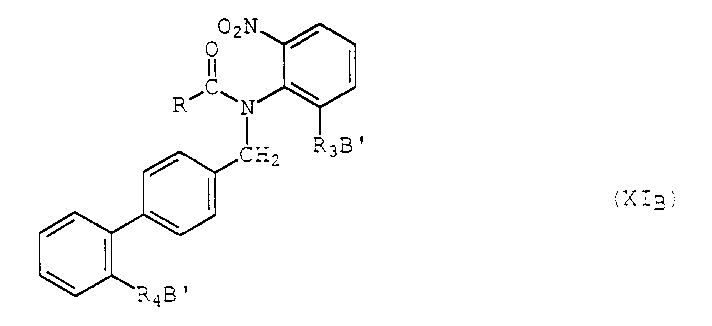 zu erhalten, in der R, R₃B' und R₄B' die vorstehenden Bedeutungen besitzen, das man einer Reaktion zur selektiven Reduktion der Nitrofunktion unterzieht, um das Produkt der Formel (XII_{B}) 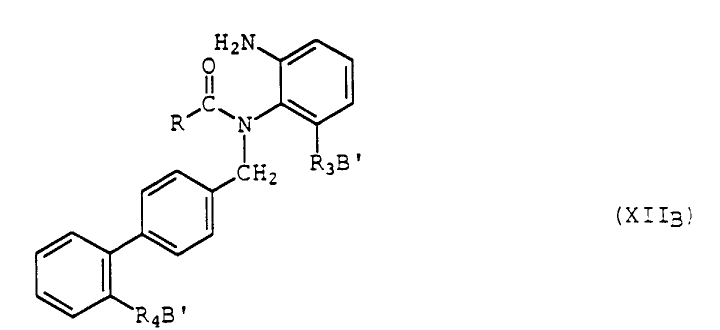 zu erhalten, in der R, R₃B' und R₄B' die vorstehenden Bedeutungen besitzen, das man einer Reaktion zur Cyclisierung unterzieht, um die wie oben definierten Produkte der Formel (IX_{B}) zu erhalten,
oder
die Verbindung der Formel (VI_{B}) mit der wie oben definierten Verbindung der Formel (V_{B}) zur Reaktion bringt, um Produkte der Formel (VII_{B}) 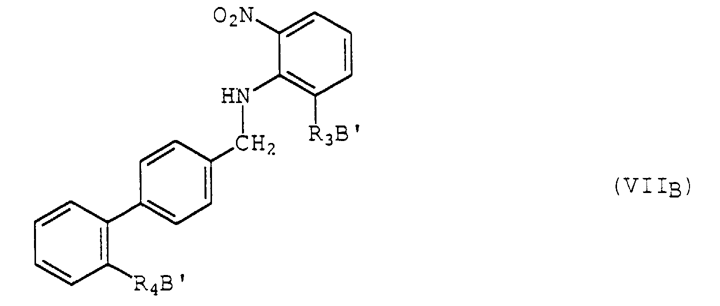 zu erhalten, in der R₃B' und R₄B' die oben angegebenen Bedeutungen besitzen, die man einer Reaktion zur Reduktion des Restes Nitro in einen Rest Amino unterzieht, um die Produkte der Formel (VIII_{B}) 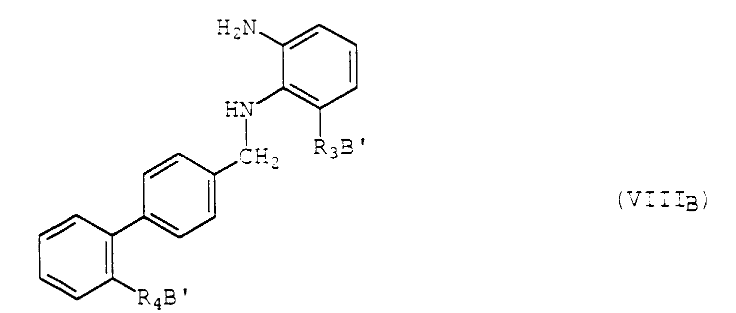 zu erhalten, in der R₃B' und R₄B' die oben angegebenen Bedeutungen besitzen, die man mit dem Produkt der Formel (III) 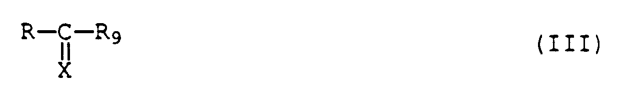 zur Reaktion bringt, in der X ein Sauerstoffatom oder ein Rest NH ist, R₉ und R die oben angegebenen Bedeutungen besitzen, um nach der Cyclisierung ein wie oben definiertes Produkt der Formel (IX_{B}) zu erhalten,
das man, wenn erwünscht und wenn erforderlich, einer oder mehreren der folgenden Reaktionen in irgendeiner Reihenfolge unterwirft:
- einer Reaktion zur Entfernung der Schutzgruppen, die die geschützten reaktiven Funktionen tragen können,
- einer Reaktion zur Salzbildung durch eine Mineralsäure oder organische Säure oder Mineralbase oder organische Base, um das entsprechende Salz zu erhalten,
- einer Reaktion zur Veresterung oder Salzbildung einer Säurefunktion,
- einer Reaktion zur sauren oder alkalischen Hydrolyse einer Esterfunktion in die Säurefunktion,
- einer Reaktion zur Aufspaltung der racemischen Formen in die Spaltprodukte,
wobei die auf diese Weise erhaltenen Produkte der Formel (I_{B}) in allen möglichen racemischen, enantiomeren oder diastereoisomeren Isomerie-Formen vorliegen.

2. Verfahren zur Herstellung von pharmazeutischen Zusammensetzungen, dadurch gekennzeichnet, daß man als Wirkstoff mindestens eines der Produkte der Formel (I_{B}) 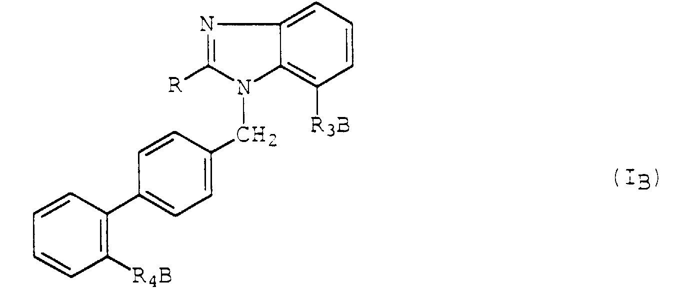 in der
R einen Rest Butyl darstellt;
R₃B einen Rest -COOR₇ bedeutet, worin R₇ ein Wasserstoffatom oder ein gerader oder verzweigter Rest Alkyl oder Alkenyl mit höchstens 5 Kohlenstoffatomen ist;
R₄B einen freien, veresterten oder in Salz überführten Rest Carboxy darstellt,
wobei die genannten Produkte der Formel (I_{B}) in allen möglichen racemischen, enantiomeren oder diastereoisomeren Isomerie-Formen vorliegen, sowie die Additionssalze der genannten Produkte der Formel (I_{B}) mit Mineralsäuren oder organischen Säuren und Mineralbasen oder organischen Basen verwendet.

3. Verfahren zur Herstellung von pharmazeutischen Zusammensetzungen nach Anspruch 2, bei dem in dem Produkt der Formel (I_{B}) R₃B COOH ist.

4. Verfahren zur Herstellung von pharmazeutischen Zusammensetzungen nach Anspruch 2, bei dem das Produkt der Formel (I_{B}) der folgenden Formel entspricht:
2-Butyl-1-[(2'-carboxy-biphenyl-4-yl)-methyl]-1H-benzimidazol-7-carbonsäure und ihre Salze.

## Claims (Claims for the following Contracting State(s): AT, BE, CH, LI, DE, DK, FR, GB, IT, LU, NL, SE)

1. Products of formula (I_{B}): 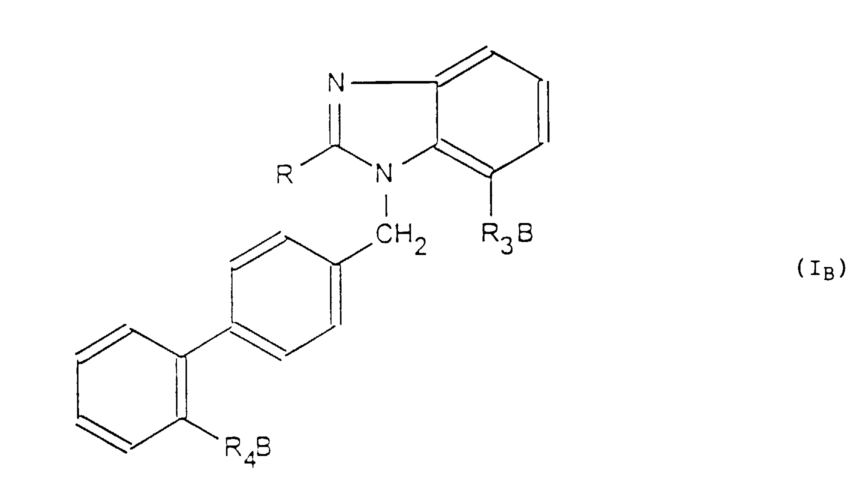 in which:
R represents a butyl radical;
R_{3B} represents a -COOR₇ radical in which R₇ represents a hydrogen atom or an alkyl or a linear or branched alkenyl radical, containing up to 5 carbon atoms;
R_{4B} represents a free esterified or salified carboxy radical; said products of formula (I_{B}) being in all the possible racemic, enantiomeric or diastereoisomeric isomer forms, as well as their addition salts with mineral or organic acids and mineral or organic bases of said products of formula (I_{B}).

2. Products of formula (I_{B}) according to claim 1, in which R_{3B} is COOH.

3. Products of formula (I_{B}) according to claim 1 or 2, corresponding to the formula:
2-butyl 1-[2'-carboxy biphenyl-4-yl) methyl] 1H-benzimidazol-7-carboxylic acid and its salts.

4. Preparation process for the products of formula (I_{B}) as defined in claim 1 characterized in that:
**either** a product of formula (IV_{B}): 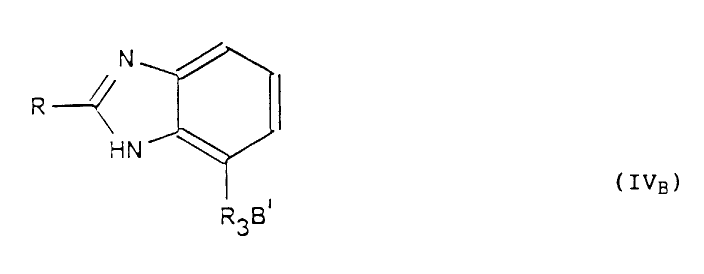 in which R and R_{3B}' have the meanings indicated in claim 1, respectively for R and R_{3B} in which optional reaction functions are optionally protected by protective groups, is reacted with a compound of formula (V_{B}): 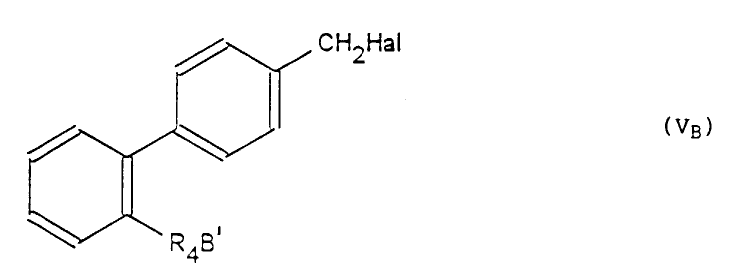 in which Hal represents a halogen atom and R_{4B}' has the meaning indicated in claim 1 for R_{4B} in which the optional reaction functions are optionally protected by protective groups, in order to obtain products of formula (IX_{B}): 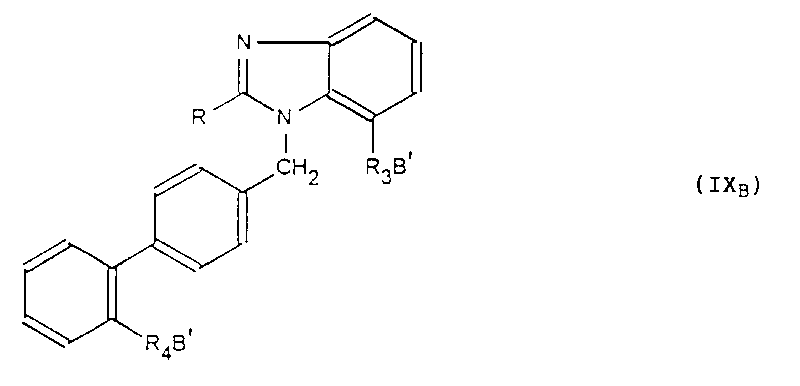 in which R, R_{3B}' and R_{4B}' have the meanings indicated above,
**or** a) a compound of formula (VI_{B}): 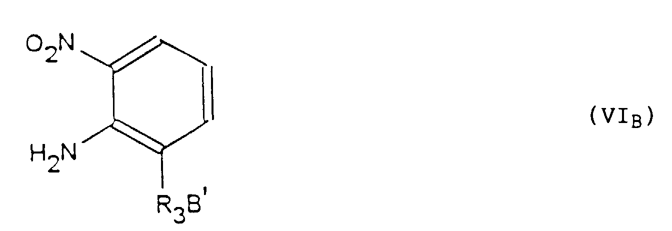 in which R_{3B}' has the meaning indicated above, is reacted either with the compound of formula (II): 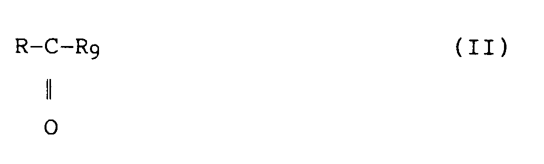 in which R₉ represents a hydroxy, alkyloxy radical or a halogen atom and R has the meaning indicated above, in order to obtain the product of formula (X_{B}): 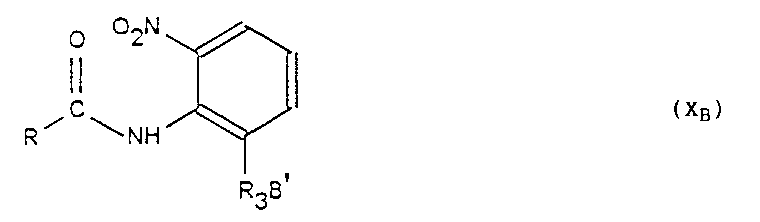 in which R and R_{3B}' have the meanings indicated above,
b) a compound of formula (VI_{B}'): 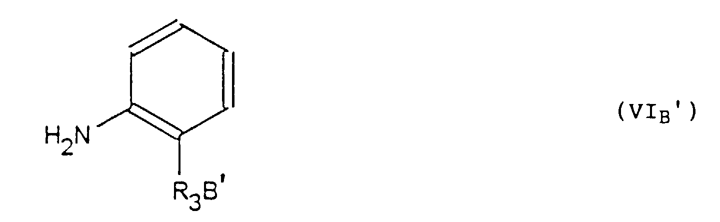 in which R_{3B}' has the meaning indicated above, is reacted with the compound of formula (II) as defined above in order to obtain the product of formula (X_{B}'): 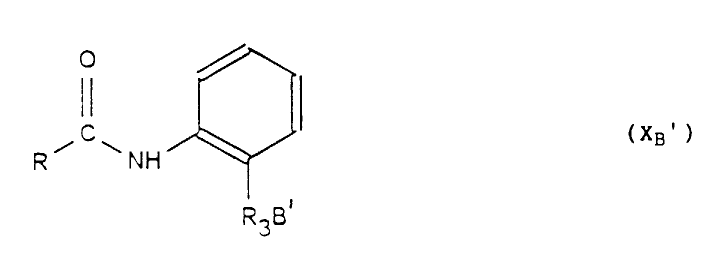 in which R_{3B}' and R have the meanings indicated above,
which is subjected to a nitration reaction in order to obtain the compound of formula (X_{B}) as defined above,
which product of formula (X_{B}) is reacted with the compound of formula (V_{B}) as defined above, in order to obtain a product of formula (XI_{B}): 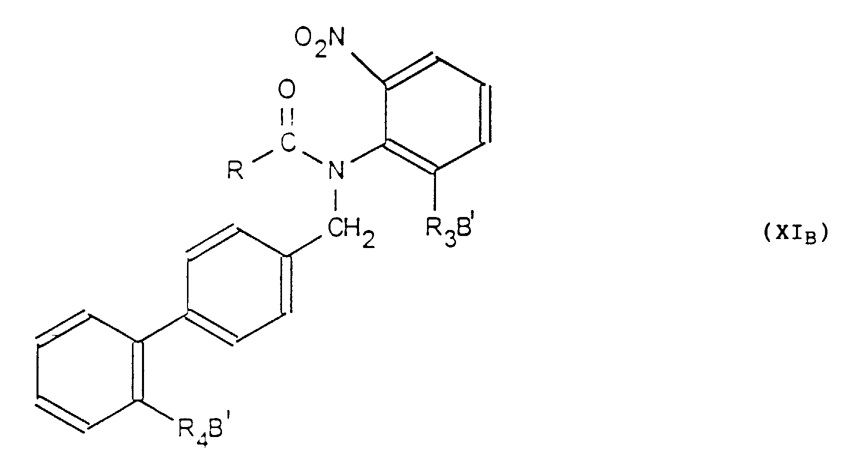 in which R, R_{3B}' and R_{4B}' have the meanings indicated above, which is subjected to a selective reduction reaction of the nitro function, to obtain the product of formula (XII_{B}): 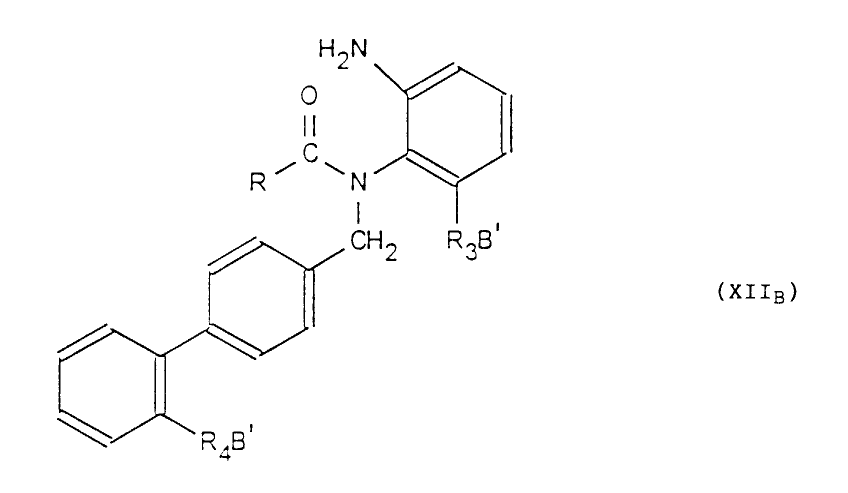 in which R, R_{3B}' and R_{4B}' have the previous meanings, which are subjected to a cyclisation reaction in order to obtain products of formula (IX_{B}) as defined above,
**or** the compound of formula (VI_{B}) is reacted with the compound of formula (V_{B}) as defined above, in order to obtain products of formula (VII_{B}): 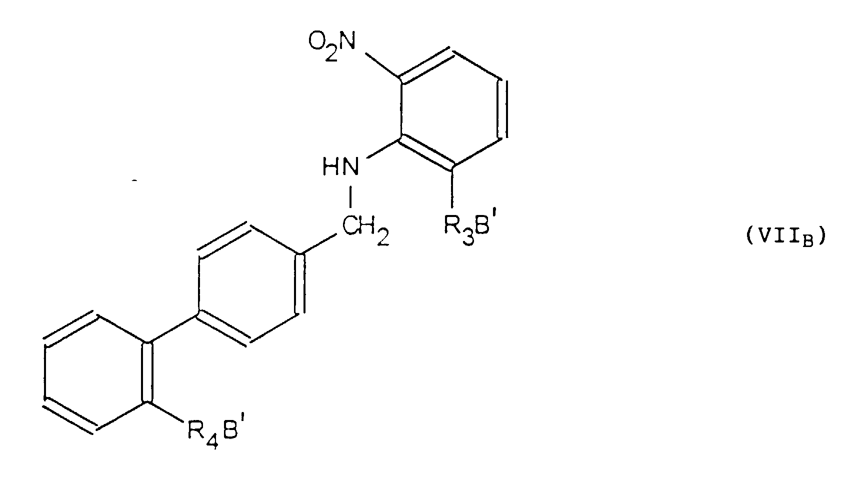 in which R_{3B}' and R_{4B}' have the meanings indicated above, which are subjected to a reaction which reduces the nitro radical to an amino radical in order to obtain products of formula (VIII_{B}): 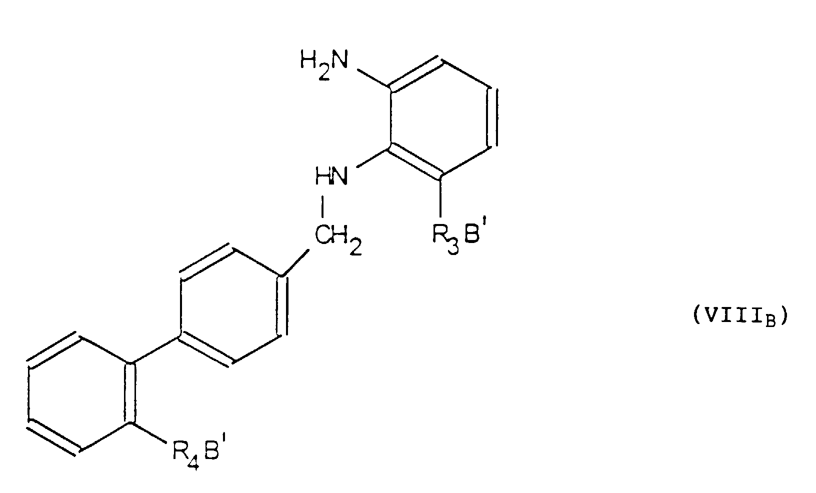 in which R_{3B}' and R_{4B}' have the meanings indicated above, which is reacted with the product of formula (III): 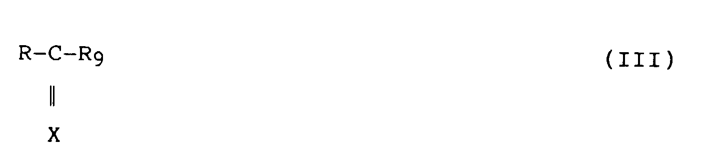 in which X represents an oxygen atom or an NH radical, R₉ and R have the meaning indicated above, in order to obtain after cyclisation a product of formula (IX_{B}) as defined above, which product of formula (IX_{B}) is subjected, if desired and if necessary, to one or more of the following reactions, in any order:
- a reaction which eliminates the protective groups which may be carried by the protected reaction functions,
- a salification reaction by an acid or a mineral or organic base in order to obtain the corresponding salt,
- an esterification or salification reaction of an acid function,
- a reaction of acid or alkaline hydrolysis of an ester function to an acid function,
- a resolution reaction of the racemic forms into resolved products,
said products of formula (I_{B}) thus obtained being in all the possible racemic, enantiomeric and diastereoisomeric isomer forms.

5. As medicaments, the products of formula (I_{B}) as defined in claims 1 to 3, said products of formula (I_{B}) being in all possible, racemic, enantiomeric and diastereoisomeric isomer forms, as well as the addition salts with pharmaceutically acceptable mineral or organic acids and mineral or organic bases of said products of formula (I_{B}).

6. The pharmaceutical compositions containing as active ingredient at least one of the medicaments as defined in claim 5.

7. The compounds of formulas (VII_{B}) and (VIII_{B}) as defined in claim 4.

8. Use of the products according to one of claims 1 to 3 for the preparation of a medicament intended for the treatment of arterial hypertension, cardiac insufficiencies, renal insufficiencies, the prevention of post-angioplastial recurrence of stenoses, the treatment of gastro-intestinal disorders and of gynaecological disorders for their relaxing effect at the level of the uterus.

## Claims (Claims for the following Contracting State(s): ES)

1. Process for the preparation of products of formula (I_{B}): 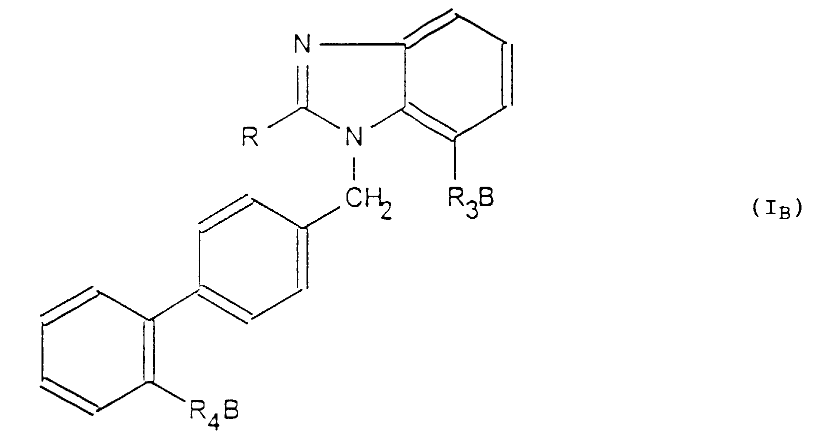 in which:
R represents a butyl radical;
R_{3B} represents a -COOR₇ radical in which R₇ represents a hydrogen atom or a linear or branched alkyl or alkenyl radical, containing up to 5 carbon atoms;
R_{4B} represents a free esterified or salified carboxy radical, said products of formula (I_{B}) being in all the possible racemic, enantiomeric or diastereoisomeric isomer forms, as well as their addition salts with mineral or organic acids and mineral or organic bases of said products of formula (I_{B}), characterized in that:
**either** a product of formula (IV_{B}): 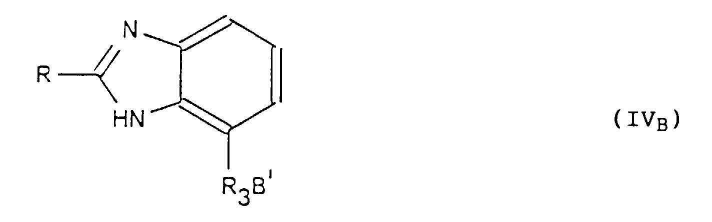 in which R and R_{3B}' have the meanings indicated above, respectively for R and R_{3B} in which optional reaction functions are optionally protected by protective groups, is reacted with a compound of formula (V_{B}): 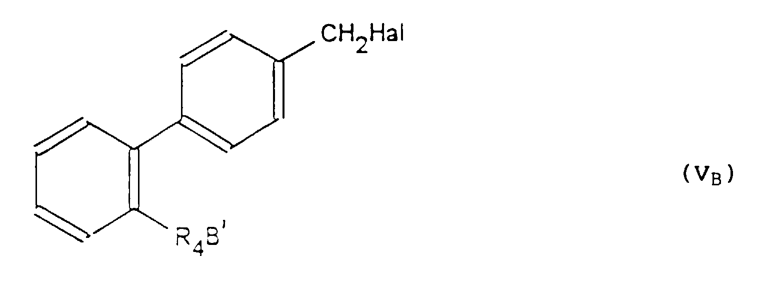 in which Hal represents a halogen atom and R_{4B}' has the meaning indicated above for R_{4B} in which the optional reaction functions are optionally protected by protective groups, in order to obtain products of formula (IX_{B}): 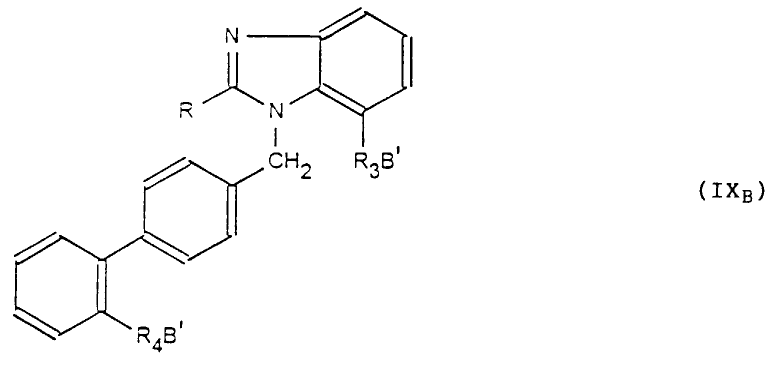 in which R, R_{3B}' and R_{4B}' have the meanings indicated above,
**or** a) a compound of formula (VI_{B}): 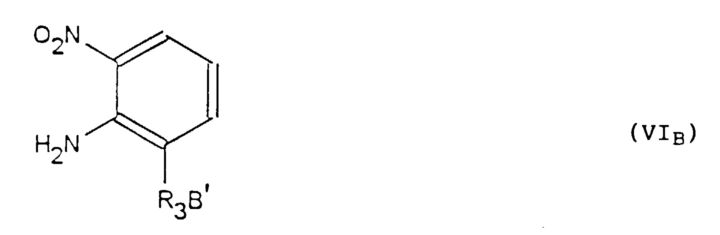 in which R_{3B}' has the meaning indicated above, is reacted either with the compound of formula (II): 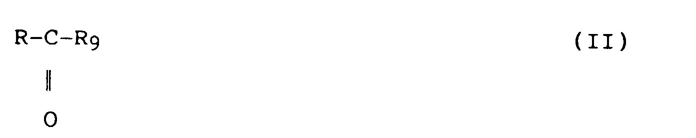 in which R₉ represents a hydroxy, alkyloxy radical or a halogen atom and R has the meaning indicated above, in order to obtain the product of formula (X_{B}): 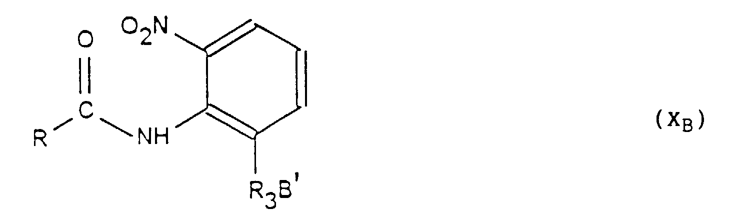 in which R and R_{3B}' have the meanings indicated above,
b) a compound of formula (VI_{B}'): 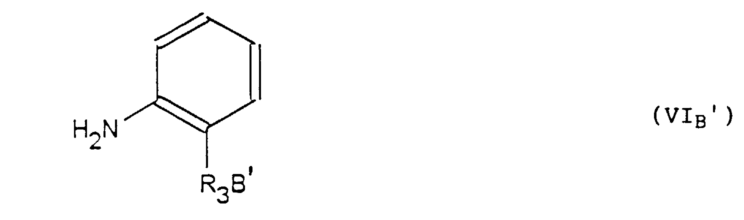 in which R_{3B}' has the meaning indicated above, is reacted with the compound of formula (II) as defined above in order to obtain the product of formula (X_{B}'): 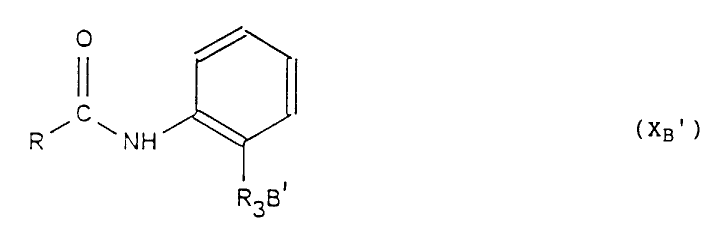 in which R_{3B}' and R have the meanings indicated above,
which is subjected to a nitration reaction in order to obtain the compound of formula (X_{B}) as defined above,
which product of formula (X_{B}) is reacted with the compound of formula (V_{B}) as defined above, in order to obtain a product of formula (XI_{B}): 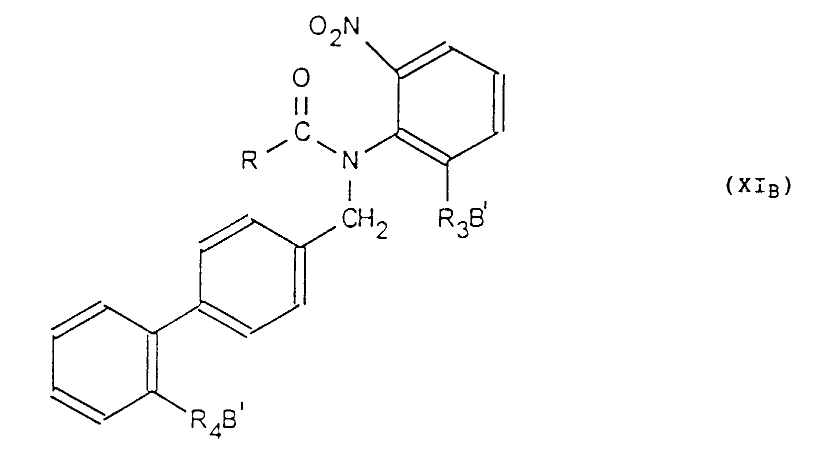 in which R, R_{3B}' and R_{4B}' have the meanings indicated above, which is subjected to a selective reduction reaction of the nitro function, in order to obtain the product of formula (XII_{B}): 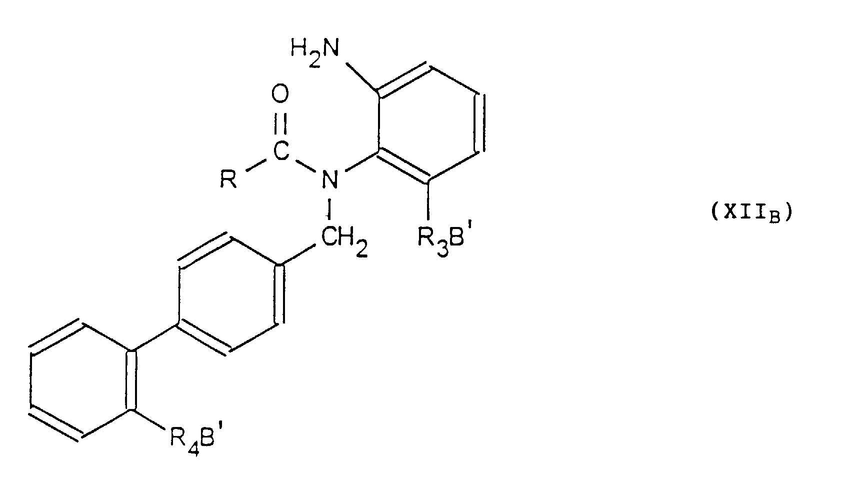 in which R, R_{3B}' and R_{4B}' have the meanings indicated above, which is subjected to a cyclisation reaction in order to obtain products of formula (IX_{B}) as defined above,
**or** the compound of formula (VI_{B}) is reacted with the compound of formula (V_{B}) as defined above, in order to obtain products of formula (VII_{B}): 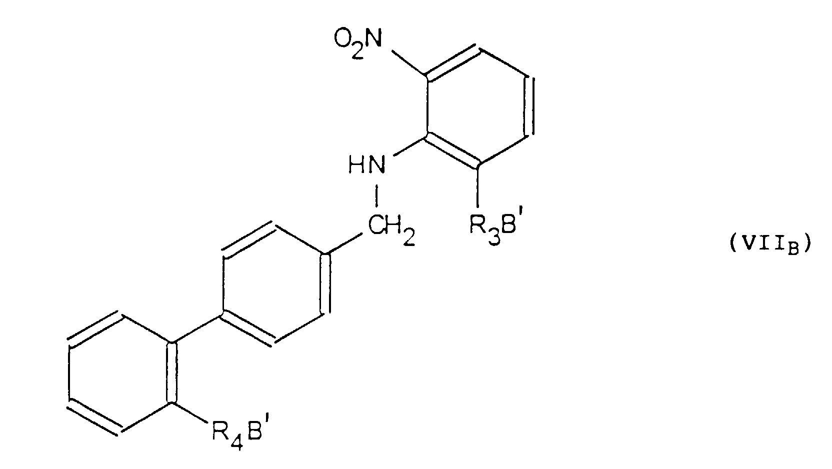 in which R_{3B}' and R_{4B}' have the meanings indicated above, which are subjected to a reaction which reduces the nitro radical to an amino radical in order to obtain products of formula (VIII_{B}): 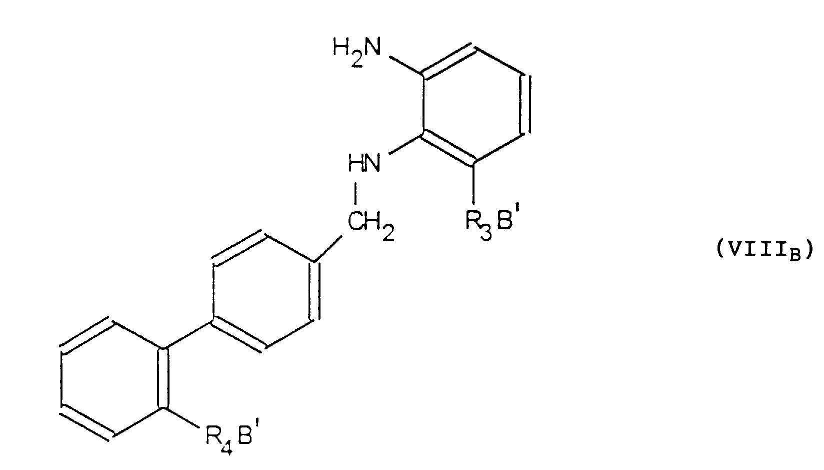 in which R_{3B}' and R_{4B}' have the meanings indicated above, which is reacted with the product of formula (III): 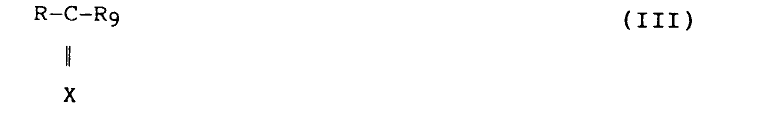 in which X represents an oxygen atom or an NH radical, R₉ and
R have the meaning indicated above, in order to obtain after cyclisation a product of formula (IX_{B}) as defined above, which product of formula (IX_{B}) is subjected, if desired and if necessary, to one or more of the following reactions, in any order:
- a reaction which eliminates the protective groups which may be carried by the protected reaction functions,
- a salification reaction by an acid or a mineral or organic base in order to obtain the corresponding salt,
- an esterification or salification reaction of an acid function,
- a reaction of acid or alkaline hydrolysis of an ester function to an acid function,
- a resolution reaction of the racemic forms into resolved products,
said products of formula (I_{B}) thus obtained being in all the possible racemic, enantiomeric and diastereoisomeric isomer forms.

## Claims (Claims for the following Contracting State(s): GR)

1. Process for the preparation of products of formula (I_{B}): 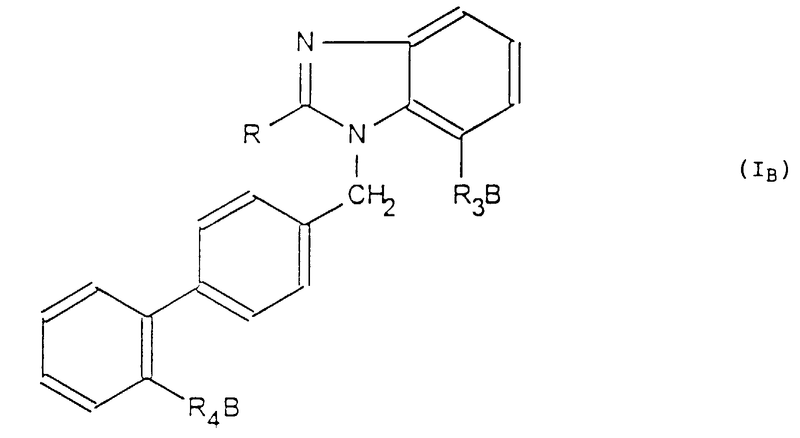 in which:
R represents a butyl radical;
R_{3B} represents a -COOR₇ radical in which R₇ represents a hydrogen atom or a linear or branched alkyl or alkenyl radical, containing up to 5 carbon atoms;
R_{4B} represents a free esterified or salified carboxy radical, said products of formula (I_{B}) being in all the possible racemic, enantiomeric or diastereoisomeric isomer forms, as well as their addition salts with mineral or organic acids and the mineral or organic bases of said products of formula (I_{B}),
characterized in that:
**either** a product of formula (IV_{B}): 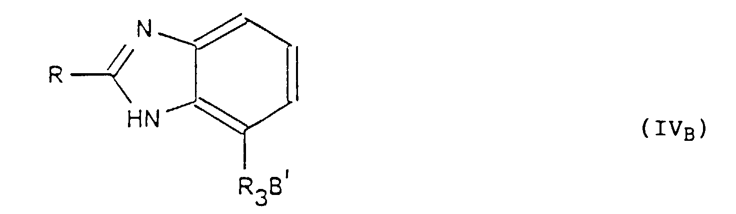 in which R and R_{3B}' have the meanings indicated above, respectively for R and R_{3B} in which optional reaction functions are optionally protected by protective groups, is reacted with a compound of formula (V_{B}): 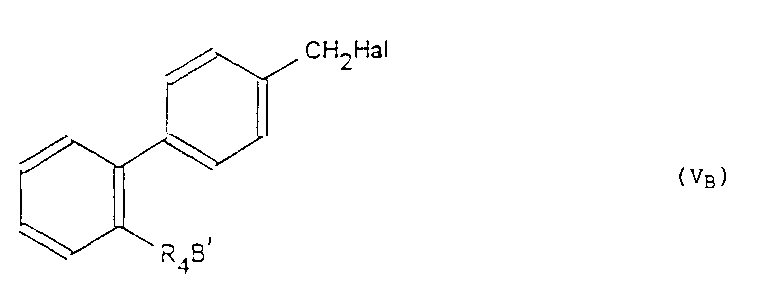 in which Hal represents a halogen atom and R_{4B}' has the meaning indicated above for R_{4B} in which the optional reaction functions are optionally protected by protective groups, in order to obtain products of formula (IX_{B}): 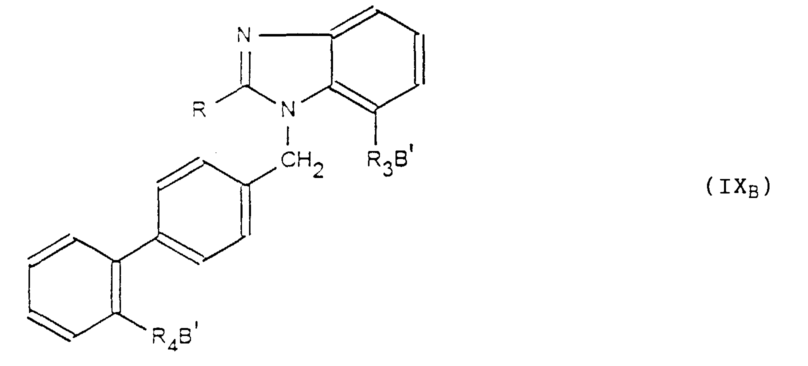 in which R, R_{3B}' and R_{4B}' have the meanings indicated above,
**or** a) a compound of formula (VI_{B}): 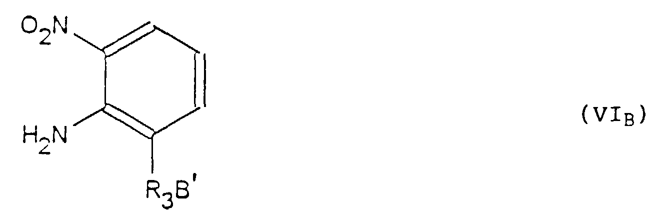 in which R_{3B}' has the meaning indicated above, is reacted either with the compound of formula (II): 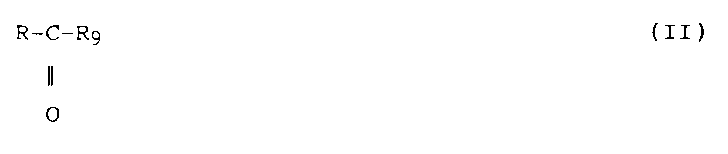 in which R₉ represents a hydroxy, alkyloxy radical or a halogen atom and R has the meaning indicated above, in order to obtain the product of formula (X_{B}): 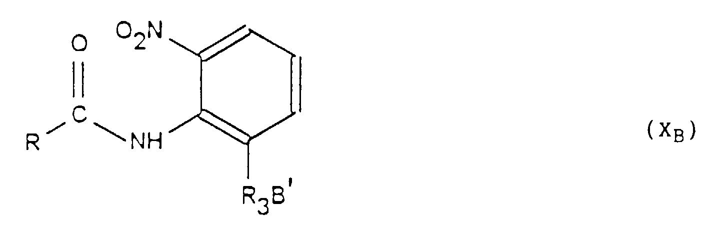 in which R and R_{3B}' have the meanings indicated above,
b) a compound of formula (VI_{B}'): 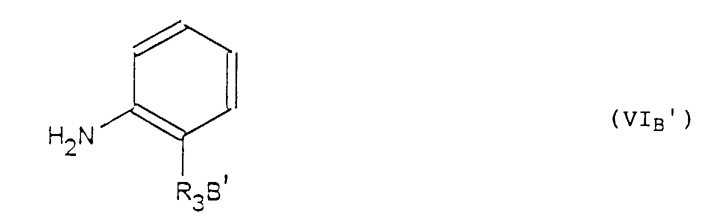 in which R_{3B}' has the meaning indicated above, is reacted with the compound of formula (II) as defined above in order to obtain the product of formula (X_{B}'): 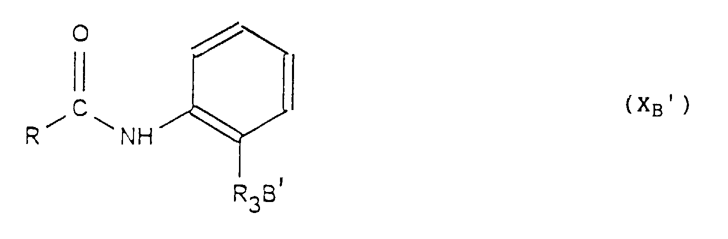 in which R_{3B}' and R have the meanings indicated above,
which is subjected to a nitration reaction in order to obtain the compound of formula (X_{B}) as defined above,
which product of formula (X_{B}) is reacted with the compound of formula (V_{B}) as defined above, in order to obtain a product of formula (XI_{B}): 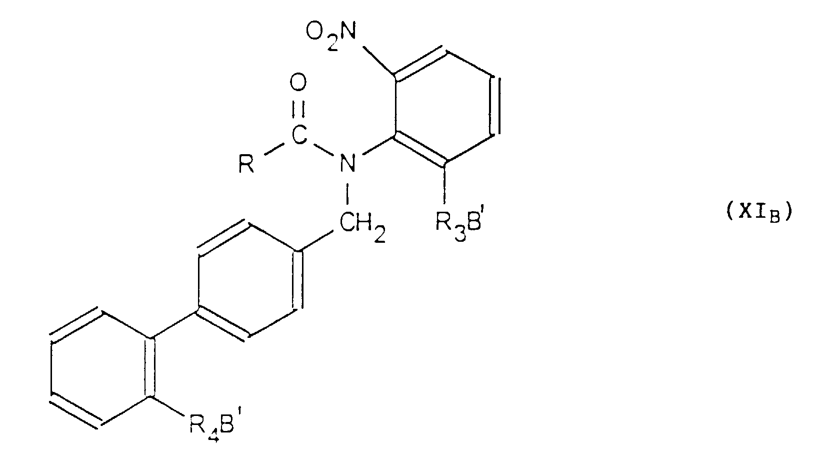 in which R, R_{3B}' and R_{4B}' have the meanings indicated above, which is subjected to a selective reduction reaction of the nitro function, in order to obtain the product of formula (XII_{B}): 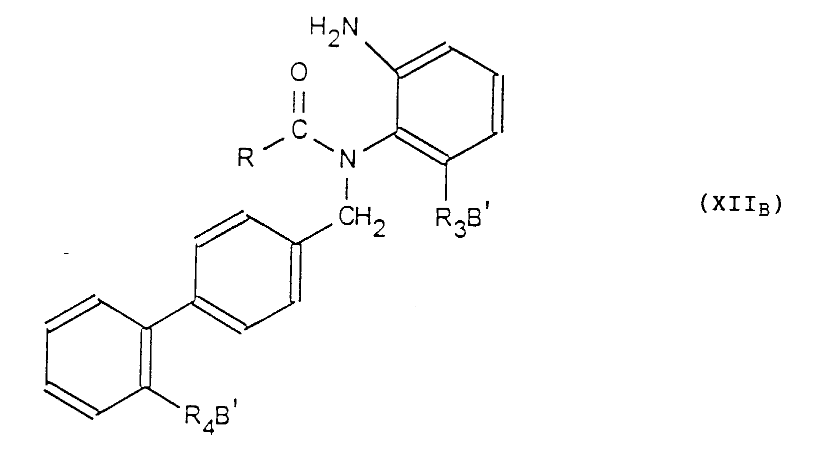 in which R, R_{3B}' and R_{4B}' have the meanings indicated above, which is subjected to a cyclisation reaction in order to obtain products of formula (IX_{B}) as defined above,
**or** the compound of formula (VI_{B}) is reacted with the compound of formula (VB) as defined above, in order to obtain products of formula (VII_{B}): 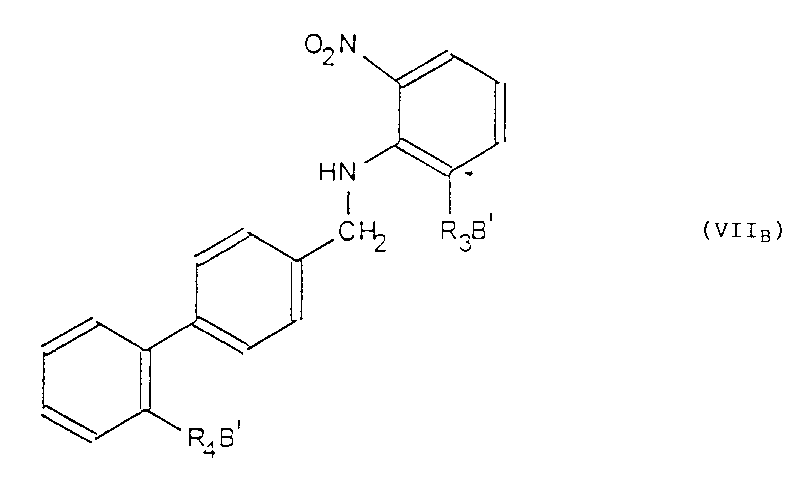 in which R_{3B}' and R_{4B}' have the meanings indicated above, which are subjected to a reaction which reduces the nitro radical to an amino radical in order to obtain products of formula (VIII_{B}): 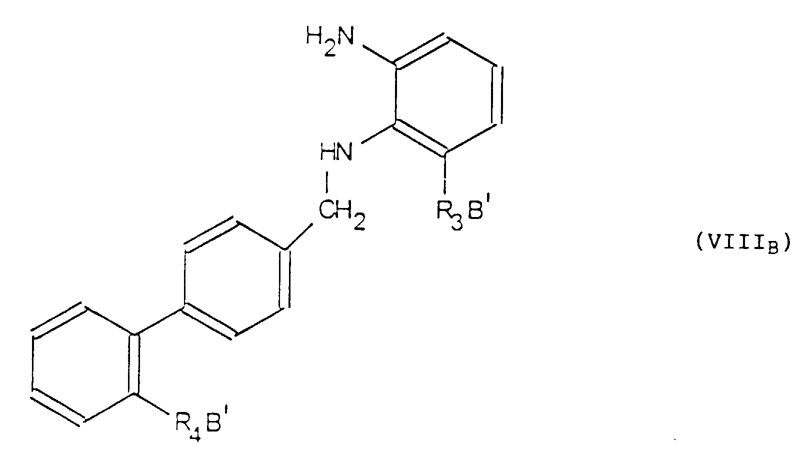 in which R_{3B}' and R_{4B}' have the meanings indicated above, which is reacted with the product of formula (III): 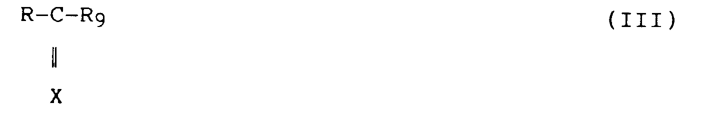 in which X represents an oxygen atom or an NH radical, R₉ and R have the meaning indicated above, in order to obtain after cyclisation a product of formula (IX_{B}) as defined above,
which product of formula (IX_{B}) is subjected, if desired and if necessary, to one or more of the following reactions, in any order:
- a reaction which eliminates the protective groups which may be carried by the protected reaction functions,
- a salification reaction by an acid or a mineral or organic base in order to obtain the corresponding salt,
- an esterification or salification reaction of an acid function,
- a reaction of acid or alkaline hydrolysis of an ester function to an acid function,
- a resolution reaction of the racemic forms into resolved products,
said products of formula (I_{B}) thus obtained being in all the possible racemic, enantiomeric and diastereoisomeric isomer forms.

2. Process for the preparation of pharmaceutical compounds characterized in that at least one of the products of formula (I_{B}): 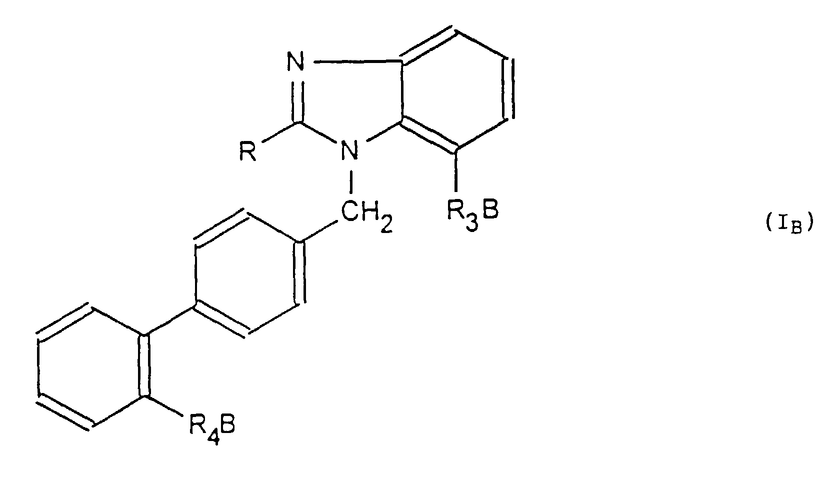 in which:
R represents a butyl radical;
R_{3B} represents a -COOR₇ radical in which R₇ represents a hydrogen atom or a linear or branched alkyl or alkenyl radical, containing up to 5 carbon atoms;
R_{4B} represents a free esterified or salified carboxy radical, said products of formula (I_{B}) being in all the possible racemic, enantiomeric or diastereoisomeric isomer forms, as well as their addition salts with mineral or organic acids and the mineral or organic bases of said products of formula (I_{B}).

3. Process for the preparation of pharmaceutical compounds according to claim 2, in which the product of formula (I_{B}) is such that R_{3B} is COOH.

4. Process for the preparation of pharmaceutical compounds according to claim 2, in which the product of formula (I_{B}) corresponds to the formula:
2-butyl 1-[2'-carboxy biphenyl-4-yl) methyl] 1H-benzimidazole-7-carboxylic acid and its salts.
